(19) Europäisches Patentamt
European Patent Office
Office européen des brevets

(11) Numéro de publication: **0 495 803 B1**

(12) # FASCICULE DE BREVET EUROPEEN

(45) Date de publication de fascicule du brevet: **10.05.95**

(51) Int. Cl.[6]: **A01N 57/34**, A01N 57/36, C02F 1/50, //(A01N57/34,59:20, 59:16,33:12),(A01N57/36,59:20, 59:16,33:12)

(21) Numéro de dépôt: **90914486.7**

(22) Date de dépôt: **09.10.90**

(86) Numéro de dépôt internationale : **PCT/BE90/00055**

(87) Numéro de publication internationale : **WO 91/04668 (18.04.91 91/09)**

(54) **COMPOSITION DESINFECTANTE ET PROCEDE DE DESINFECTION.**

(30) Priorité: **09.10.89 BE 8901081**

(43) Date de publication de la demande: **29.07.92 Bulletin 92/31**

(45) Mention de la délivrance du brevet: **10.05.95 Bulletin 95/19**

(84) Etats contractants désignés: **BE DE ES FR IT**

(56) Documents cités:
**EP-A- 0 258 737**
**WO-A-87/02221**
**US-A- 4 835 143**

(73) Titulaire: **FABRICOM AIR CONDITIONING S.A.**
**rue du Montenégro, 138 à 144**
**B-1060 Bruxelles (BE)**

(72) Inventeur: **LEGROS, Alain**
**Rue du Docteur-Maître 11**
**B-6120 Nalinnes (BE)**

(74) Mandataire: **Vanderperre, Robert et al**
**Bureau Vander Haeghen S.A.**
**Rue Colonel Bourg 108 A**
**B-1040 Bruxelles (BE)**

**EP 0 495 803 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à une composition destinée à la désinfection de liquides et/ou de surfaces ou à la conservation d'aliments ou boissons, cette composition contenant au moins un composé de phosphonium quaternaire et au moins un composé d'ammonium quaternaire de formule (I) ayant un poids moléculaire compris entre 1000 et 50.000, de préférence entre 1000 et 5000.

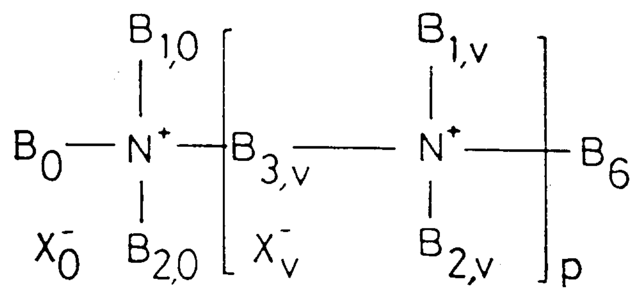

(I)

L'invention est également relative à un procédé de désinfection utilisant une telle composition.

## L'ETAT DE LA TECHNIQUE

Il est connu de désinfecter des liquides et/ou des surfaces au moyen de compositions contenant des composés d'ammonium quaternaire.

Ainsi le document WO-A-87/02221 décrit des compositions contenant un polymère ou copolymère obtenu par condensation d'une diamine avec un dihalogénure et des ions d'au moins un métal choisi parmi le cuivre, l'argent et le manganèse.

Il est également connu d'utiliser, par exemple, par le document US-A-3 364 141, des composés de phosphonium pour le traitement d'eau industrielle, ces composés agissant en tant qu'algicides, fongicides et bactéricides.

Bien que les solutions proposées dans ces documents sont acceptables, l'homme du métier a toujours essayé de réduire les quantités de désinfectants nécessaires pour obtenir un même résultat de désinfection.

Ainsi le document EP-A-0 258 737 enseigne une composition désinfectante contenant un phosphonium et un ammonium, à savoir du benzyl-diméthyl-alkyl ammonium, le rapport en poids entre l'ammonium et le phosphonium étant compris entre 1:9 et 9:1.

Si cette composition semble être efficace à l'encontre de germes de souches cibles, il semble qu'elle ne soit que peu active pour tuer des germes de souches stressées ou des germes banaux.

Le demandeur a remarqué qu'en utilisant des compositions désinfectantes contenant un phosphonium et des ammoniums particuliers, il était possible de tuer rapidement des germes banaux et des germes stressés.

## DESCRIPTION DE L'INVENTION

La composition désinfectante suivant l'invention qui peut être utilisée pour la désinfection de liquides et/ou surfaces ou pour la conservation d'aliments ou boissons contient au moins un composé d'ammonium quaternaire, ayant un poids moléculaire compris entre 1000 et 50.000 de formule (I)

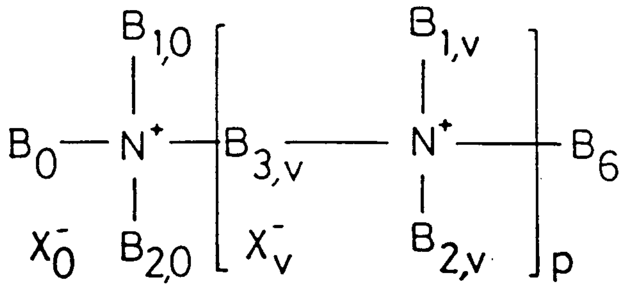

(I)

dans laquelle :

- p est un nombre entier au moins égal à 1 ;
- v est un nombre entier compris entre 1 et p ;
- $B_O$ et $B_6$ qui peuvent être identiques ou différents désignent un radical hydrocarboné éventuellement insaturé, ce radical contenant de 1 à 22 atomes de carbone ;
- $B_{1,0}$, $B_{2,0}$, $B_{1,v}$ et $B_{2,v}$ pour v compris entre 1 et p désignent un groupe hydrocarboné éventuellement ramifié, insaturé et/ou substitué par un ou plusieurs halogènes ou par un groupement hydrocarboné ou par un groupement carboxyle ou par un groupement hydroxyle, $B_{1,0}$, $B_{2,0}$, $B_{1,v}$ et $B_{2,v}$ pouvant contenir jusqu'à 22 atomes de carbone, $B_{1,v}$ et/ou $B_{2,v}$ pouvant être reliés respectivement à $B_{1,v+1}$ et/ou $B_{1,v-1}$ et à $B_{2,v+1}$ et/ou $B_{2,v-1}$ ;
- $B_{3,v}$ pour v compris entre 1 et p désigne un groupe hydrocarboné éventuellement substitué, insaturé et/ou ramifié pouvant contenir jusqu'à 20 atomes de carbone ou un groupe biguanide ou un groupe de formule

(1) o-, m- ou p-xylydène de formule :

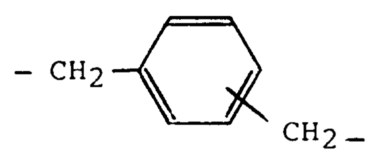

(2)

$$-(CH_2)_y-\underset{E}{\underset{|}{CH}}-(CH_2)_x-\underset{K}{\underset{|}{CH}}-(CH_2)_t-$$

où x, y et t sont des nombres entiers allant de 0 à 11, tels que la somme de x + y + t est égale ou supérieure à 0 et inférieure à 18, tandis que les symboles E et K désignent l'hydrogène ou des radicaux alkyle contenant moins de 18 atomes de carbone ;

(3) $-(CH_2)_n-S-(CH_2)_n-$ ,
(4) $-(CH_2)_n-O-(CH_2)_n-$ ,
(5) $-(CH_2)_n-S-S-(CH_2)_n-$ ,
(6) $-(CH_2)_n-SO-(CH_2)_n-$ ,
(7) $-(CH_2)_n-SO_2-(CH_2)_n-$ ,
(8)

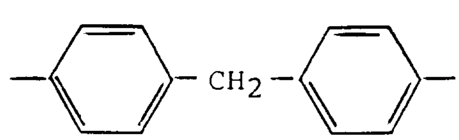

où n est égal à 1, 2 ou 3.

(9)

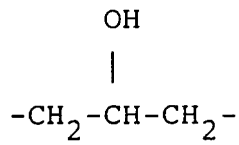

et

$X^-_0$ et $X^-_{v+1}$ désignent un anion, de préférence un atome d'halogène tel qu'un atome de brome ou de chlore,

et au moins un composé de formule II

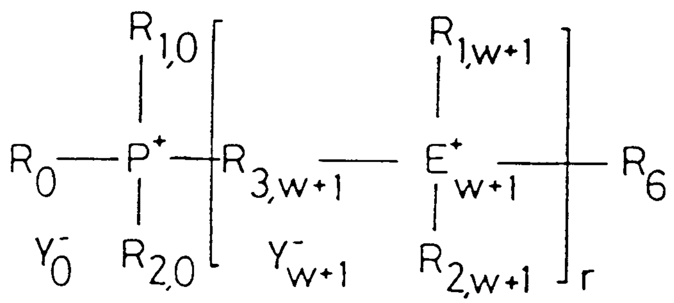

(II)

dans laquelle :

- r est un nombre entier éventuellement égal à 0 ;
- w est un nombre entier compris entre 0 et r ;
- $R_0$ et $R_6$ qui peuvent être identiques ou différents désignent un radical hydrocarboné éventuellement substitué et éventuellement insaturé, ce radical contenant de 1 à 22 atomes de carbone ;
- $R_{1,0}$ , $R_{2,0}$ , $R_{1,w+1}$ et $R_{2,w+1}$ pour w compris entre 0 et r désignent un groupe hydrocarboné éventuellement ramifié, insaturé et/ou substitué par un ou plusieurs halogènes ou par un groupement hydrocarboné ou par un groupement hydroxyle, $R_{1,w+1}$ et $R_{2,w+1}$ pouvant contenir jusqu'à 22 atomes de carbone, $R_{1,w+1}$ et/ou $R_{2,w+1}$ pouvant être reliés respectivement à $R_{1,w+2}$ et/ou $R_{1,w}$ et à $R_{2,w+2}$ et/ou $R_{2,w}$ ;
- $E^+_{w+1}$ étant choisi pour w compris entre 0 et r parmi N et P ;
- $R_{3,w+1}$ désigne un groupe hydrocarboné éventuellement insaturé et/ou ramifié pouvant contenir jusqu'à 20 atomes de carbone ou un groupe de formule :

(1) o-, m- ou p-xylylidène de formule :

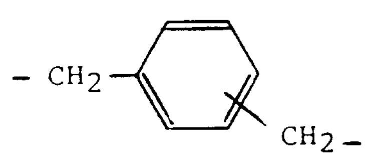

(2)

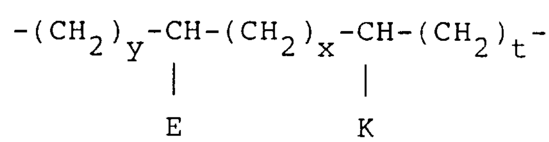

où x, y et t sont des nombres entiers allant de 0 à 11, tels que la somme de x + y + t est égale ou

supérieure à 0 et inférieure à 18, tandis que les symboles E et K désignent de l'hydrogène ou des radicaux alkyle contenant moins de 18 atomes de carbone ;

(3) $-(CH_2)_n-S-(CH_2)_n-$ ,

(4) $-(CH_2)_n-O-(CH_2)_n-$ ,

(5) $-(CH_2)_n-S-S-(CH_2)_n-$ ,

(6) $-(CH_2)_n-SO-(CH_2)_n-$ ,

(7) $-(CH_2)_n-SO_2-(CH_2)_n-$ ,

(8)

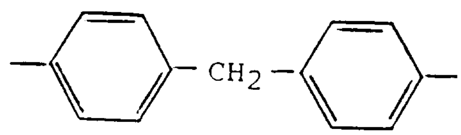

où n est égal à 1, 2 ou 3.

(9)

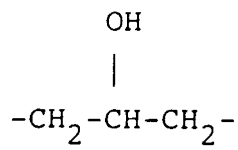

et

$Y^-_0$ et $Y^-_{w+1}$ désignent un anion, de préférence un halogène. De préférence, le poids moléculaire est un composé d'ammonium quaternaire et compris entre 1000 et 5000.

La composition suivant l'invention contient également avantageusement un ion d'un métal choisi avantageusement parmi le fer, le cuivre, l'argent, l'or, le manganèse, le zinc et un mélange de tels ions, cet ion de métal permettant d'accroître encore l'efficacité de la composition.

De préférence, la composition contient de 5 à 95 % de composé de formule II par rapport à la quantité totale de composé de formule I et de composé de formule II présents dans la composition.

Selon des particularités des composés de formules I et II, $R_O$ et/ou $R_6$ et/ou $B_O$ et/ou $B_6$ sont des groupes hydrocarbonés éventuellement substitués et insaturés contenant de 16 à 22 atomes de carbone, de préférence 16 atomes de carbone. Ces groupes peuvent, par exemple, être substitués par un ou des groupes hydroxyles ou carboxyles ou par des halogènes.

Dans des formes particulières des composés de formules (I) et (II),

- pour au moins un l compris entre 0 et p, $B_{1,l}$ et $B_{1,l+1}$ sont reliés entre eux et/ou $B_{2,l}$ et $B_{2,l+1}$ sont reliés entre eux en formant de préférence un groupe identique à $B_{3,l+1}$, tel qu'un groupe de formule $-(CH_2)_2-$, et
- pour au moins un k compris entre 0 et r, $R_{1,k}$ et $R_{1,k+1}$ sont reliés entre eux et/ou $R_{2,k}$ et $R_{2,k+1}$ sont reliés entre eux en formant de préférence un groupe identique à $R_{3,k+1}$ tel qu'un groupe de formule $-(CH_2)_2-$.

Dans d'autres formes particulières des composés de formules (I) et (II),

- $B_{3,l}$ est un groupe de formule $-CH_2-CH_2-CH_2-$ lorsque l est un nombre pair, tandis que $B_{3,l}$ est un groupe de formule $-CH_2-CH_2-CH_2-CH_2-CH_2-CH_2-$ lorsque l est un nombre impair, ou inversément, et
- $R_{3,k+1}$ est un groupe de formule $-CH_2-CH_2-CH_2-$ lorsque k est un nombre impair, tandis que $R_{3,k+1}$ est un groupe de formule $-CH_2-CH_2-CH_2-CH_2-CH_2-CH_2-$ lorsque k est un nombre pair, ou inversément.

$R_{3,k+1}$ et $B_{3,l}$ dans les composés de formules (I) et (II) peuvent, par exemple, être choisis parmi les groupes de formule

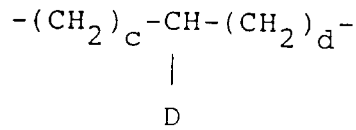

dans laquelle D désigne un atome d'hydrogène ou un radical $C_{1-4}$ alkyle, c et d sont des nombres entiers inférieurs à 5 dont l'un peut avoir une valeur égale à 0, tandis que la somme c + d est au moins égale à 1 et au maximum égale à 8.

La présente invention a également pour objet

- un procédé de désinfection de liquides dans lequel on ajoute à ces liquides au moins une composition suivant l'invention ;
- un procédé de désinfection de surfaces dans lequel on met lesdites surfaces en contact avec au moins une composition suivant l'invention ;
- un procédé de conservation de boissons dans lequel on ajoute auxdites boissons une composition suivant l'invention, et
- un procédé de conservation d'aliments dans lequel on trempe lesdits aliments dans une composition suivant l'invention.

EXEMPLES DE COMPOSES POUR DES COMPOSITIONS SUIVANT L'INVENTION

Des composés de phosphonium qui peuvent être utilisés dans les compositions suivant l'invention sont des composés de formule (II)

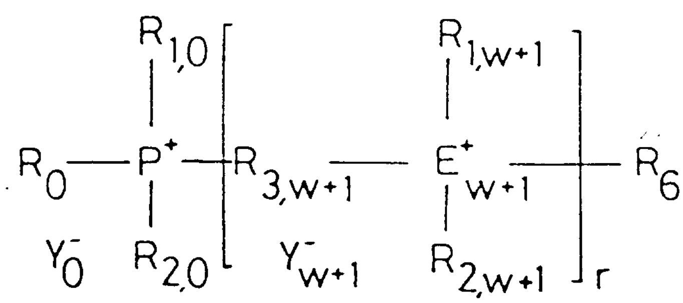

(II)

dans laquelle r, w, $R_0$ , $R_6$ , $R_{1,0}$ , $R_{2,0}$ , $R_{1,w+1}$ , $R_{2,w+1}$ , $Y^-{}_0$ , $Y^-{}_{w+1}$ ont les significations données ci-avant.

Des exemples particuliers de composés de phosphonium quaternaire sont donnés ci-après :

- des composés de formule

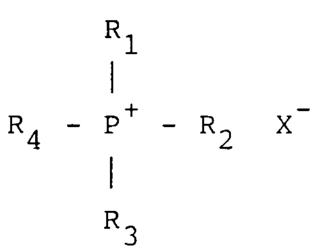

(III)

dans laquelle au moins un des symboles $R_1$, $R_2$, $R_3$, $R_4$ désigne un radical alkyle contenant plus de 6 atomes de carbone et le ou les autres symboles $R_1$, $R_2$, $R_3$ et $R_4$ désignent un radical alkyle contenant de 1 à 6 atomes de carbone ou un radical aryle monocyclique éventuellement substitué, par exemple par un radical alkyle inférieur, par un halogène ou par un groupe hydroxy, tandis que X désigne un anion choisi parmi le chlore, le brome, l'iode, le bicarbonate ou le phosphate ;

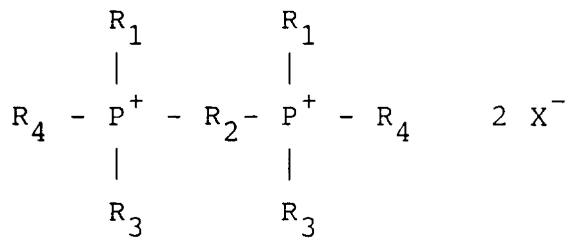

(IV)

dans laquelle les symboles $R_1$, $R_2$, $R_3$, $R_4$ et X ont les significations indiquées plus haut.

- des composés de formule

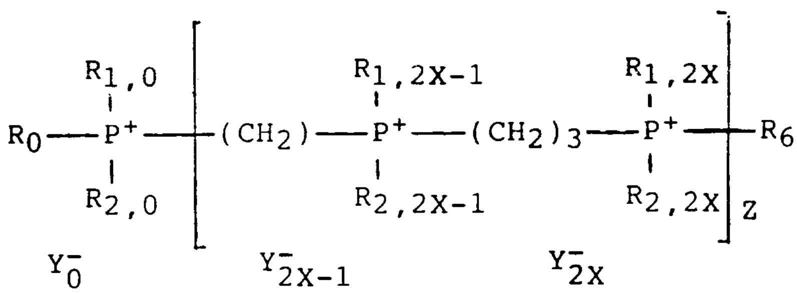

(V)

avec X compris entre 1 et Z

dans laquelle $R_0$ , $R_6$ , $R_{1,0}$ , $R_{2,0}$ , $R_{1,2X-1}$ , $R_{2,2X-1}$ $R_{1,2X}$ et $R_{2,2X}$ désignent un radical hydrocarboné éventuellement substitué et éventuellement insaturé, ce radical contenant de 1 à 22 atomes de carbone, ou encore

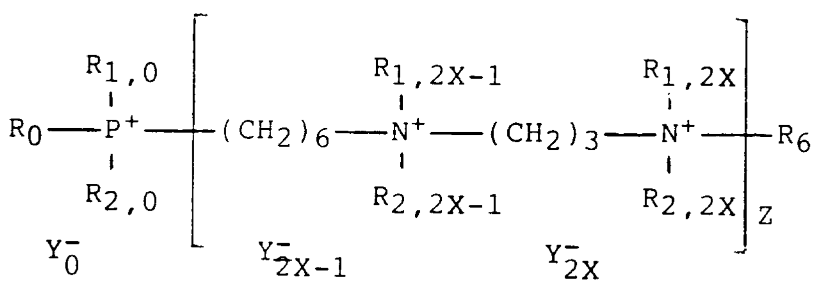

(VI)

dans laquelle $R_0$ , $R_6$ , $R_{1,0}$ , $R_{2,0}$ , $R_{1,2X-1}$ , $R_{2,2X-1}$ , $R_{1,2X}$ et $R_{2,2X}$ désignent un groupe hydrocarboné éventuellement ramifié, insaturé et/ou substitué par un ou plusieurs halogènes ou par un groupement hydrocarboné ou par un groupement hydroxyle.

Des composés de phosphonium de formule (III) peuvent être préparés en faisant réagir une phosphine avec un halogénure d'un composé hydrocarboné.

Ainsi le bromure de triphényl hexadécyl phosphonium (P2) a été préparé de la manière suivante :

On a mélangé 13,11 g de triphénylphosphine à 15,26 g de bromure d'hexadécane. Le réacteur a été maintenu à 110 °C sous atmosphère d'argon et sous agitation.

Après 24 heures de réaction, le mélange était limpide et contenait plus de 90% de bromure d'hexadécyl triphényl phosphonium.

De manière similaire, mais en faisant réagir de la triphényl phosphine ou de la diméthyl phényl phosphine ou de la diéthyl phényl phosphine ou de la tripropyl phosphine ou de la diphényl hexadécyl phosphine ou de la diphényl octyl phosphine ou de la tritolyl phosphine ou de la diphényl tolyl phosphine ou de la méthyl dioctyl phosphine avec un chlorure, bromure ou iodure de dihydroxyoctodécane, d'hexadécane, d'octadécane, de docosane, d'octane, de dodécane, de toluène ou de benzène, on a préparé les phosphonium suivants :

- bromure de triphényl hexadécyl phosphonium (P2),
- bromure de triphényl octadécyl phosphonium (P5),

- bromure de triphényl dodécyl phosphonium (P1),
- bromure de triéthyl dodécyl phosphonium,
- bromure de diméthyl dodécyl phényl phosphonium,
- chlorure de triphényl benzyl phosphonium,
- chlorure de tributyl benzyl phosphonium (P27),
- chlorure de diméthyl dodécyl phényl phosphonium,
- bromure de diéthyl dodécyl phényl phosphonium,
- bromure de tripropyl dodécyl phosphonium,
- bromure de tripropyl hexadécyl phosphonium (P3),
- bromure de diphényl dihexadécyl phosphonium (P4),
- bromure de triphényl docosyl phosphonium (P6),
- bromure de dodécyl tritolyl phosphonium (P13),
- bromure de dodécyl diphényl tolyl phosphonium (P12),
- bromure de cétyl triphényl phosphonium,
- bromure de méthyl dioctyl phényl phosphonium,
- iodure de triéthyl dodécyl phosphonium,
- bromure d'octadécyl α,w bis-triphényl phosphonium (P9),
- bromure d'hexadécyl α,w bis-triéthyl phosphonium (P7),
- bromure de diphényl dioctyl phosphonium (P10),
- bromure de triphényl dihydroxyoctadécyl phosphonium (P11),
- bromure de tripropyl benzyl phosphonium (P32).

Des composés contenant plusieurs groupements phosphonium en un ou des groupements phosphonium et un ou des groupements ammonium peuvent être obtenus en faisant réagir des phosphines avec des dihalogénures, des amines avec des dihalogénures avant de les faire réagir avec des phosphines.

A titre d'exemple on a préparé un composé P34 de formule

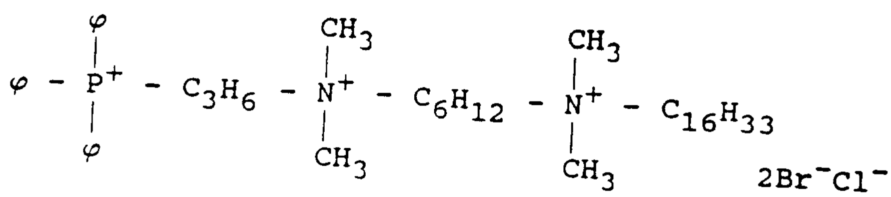

ϕ = phényle
de la manière suivante :

On a mélangé 26,2 g de triphényl phosphine avec 15,75 g de 1,3-bromochloropropane dans 100 g d'eau. On a porté le mélange à 65 °C et on a maintenu ce mélange à cette température et sous agitation pendant 3 heures.

On a ensuite ajouté 17,23 g de 1,6- tétraméthylhexane diamine.

On a porté ce mélange à 121 °C sous atmosphère d'argon et on a maintenu celui-ci à cette température et sous agitation pendant 16 heures.

On a enfin ajouté 30,5 g de 1-bromohexadécane et on a chauffé le mélange sous agitation à 90 °C pendant 12 heures.

Le produit obtenu avait un poids moléculaire de 897,38 g.

Le composé P8 de formule :

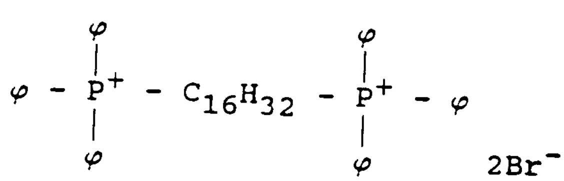

ϕ = phényle

a été préparé de la manière suivante :

On a mélangé 5,24 g de triphényl phosphine avec 3,83 g de dibromo hexadécane sous atmosphère d'argon. On a porté ce mélange à une température de 120 ° C et on a maintenu cette température pendant 4 heures.

Le composé P14 de formule :

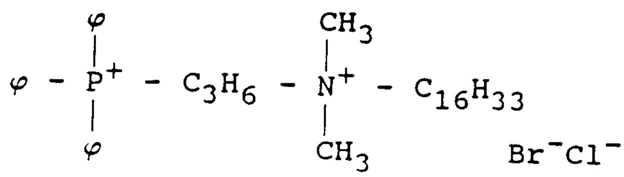

ϕ = phényle

a été obtenu de la manière suivante :

Dans un premier temps, on a synthétisé sous atmosphère d'argon un composé intermédiaire en mélangeant 26,3 g de triphényl phosphine avec 15,75 g de bromochloropropane et en maintenant la température de ce mélange à 120 ° C pendant 4 heures.

Ensuite, on a fait réagir pendant 20 heures à une température de 90 ° C 2,62 g dudit composé intermédiaire avec 1,68 g de diméthyl hexadécyl amine.

Le composé P15 de formule

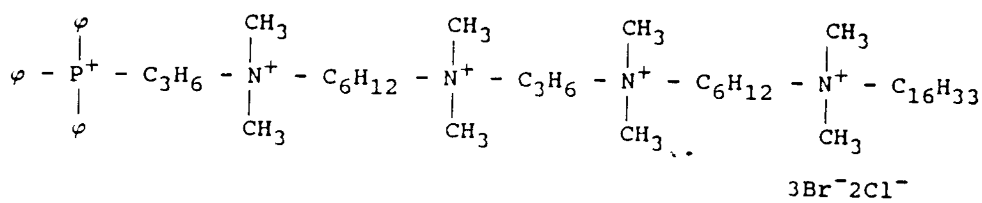

ϕ = phényle

a été préparé de la manière suivante :

On a synthétisé sous atmosphère d'argon un composé intermédiaire de phosphonium en mélangeant 26,3 g de triphényl phosphine avec 15,75 g de bromochloropropane et en portant ce mélange a 120 ° C pendant 4 heures.

On a synthétisé en milieu aqueux un composé d'ammonium en faisant réagir pendant 4 heures à 60 ° C 10,34 g de N,N,N',N'-tétraméthyl hexane diamine avec 4,72 g de bromochloropropane.

On a ensuite ajouté à ce mélange aqueux 9,16 g de bromohexadécane et on a maintenu ce mélange à une température de 90 ° C pendant 20 heures.

On a ainsi obtenu un mélange aqueux contenant un composé P15 de formule donnée ci-avant de poids moléculaire de 1227.

Le composé P16 de formule

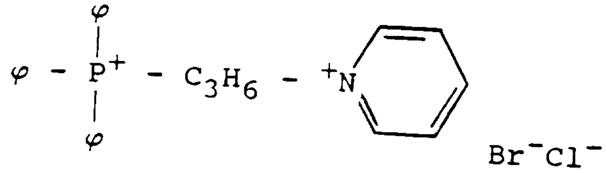

ϕ = phényle

a été préparé de la manière suivante :

Sous atmosphère d'argon, on a synthétisé un composé intermédiaire en mélangeant 26,3 g de triphényl phosphine avec 15,74 g de bromochloropropane à une température de 120 ° C pendant 4 heures. On a ensuite ajouté à ce mélange 0,99 g de pipéridine et on a maintenu la température à 60 ° C pendant 2 heures.

Le composé P17 de formule

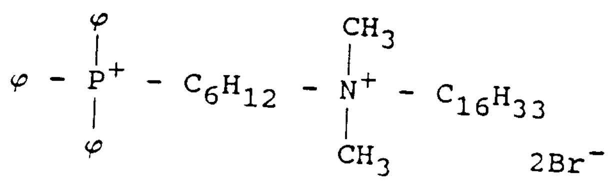

$\phi$ = phényle

a été préparé de la manière suivante :

On a fait réagir pendant 2 heures à 60 ° C 26,95 g de diméthylhexadécyl amine avec 24,4 g de 1,6-dibromohexane. On a ensuite fait réagir 5,14 g du produit de la première réaction avec 2,62 g de triphényl phosphine à une température de 120 ° C pendant 2 heures sous atmosphère d'argon.

Le composé P20 de formule

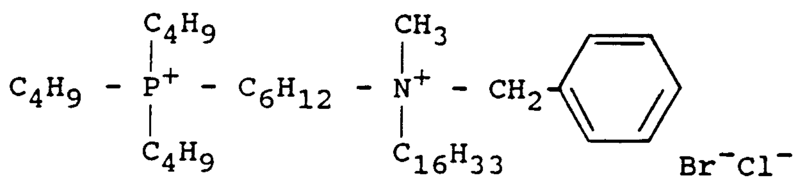

a été préparé en préparant un composé intermédiaire par réaction pendant 20 heures à 130 ° C sous atmosphère d'argon de 20,23 g de tributyl phosphine avec 19,95 g de 1-bromo-6-chloro hexane et en faisant réagir 6,03 g du composé intermédiaire avec 5,18 g de benzylméthyl hexadécyl amine pendant 20 heures à 90 ° C.

Le composé P21 de formule

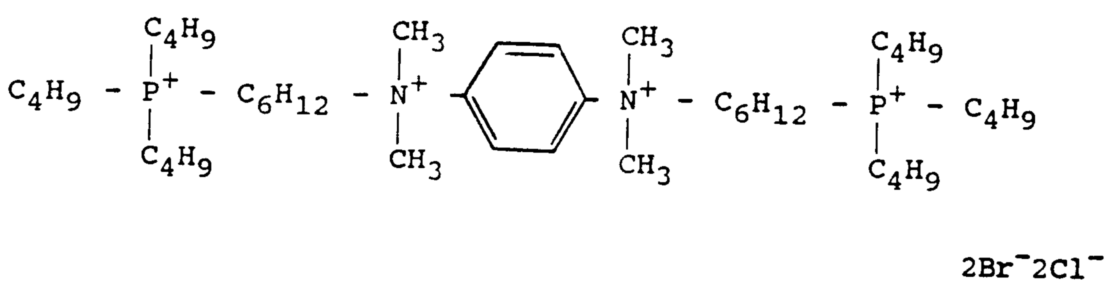

a été obtenu en faisant réagir 6,03 g du composé intermédiare obtenu lors de la préparation du composé P20 avec 1,23 g de N,N,N',N'-tétraméthyl-p-phénylène diamine pendant 20 heures à 90 ° C.

Le composé P22 de formule

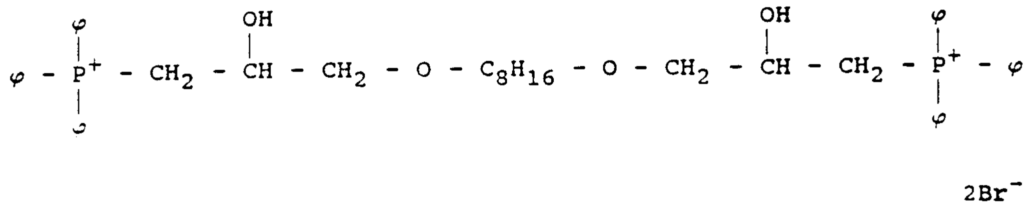

$\phi$ = phényle

a été préparé de la manière suivante :

On a fait réagir pendant 3 heures à 120 °C 7,31 g de 1,8-octanediol avec 13,7 g d'épibromhydrine. Après filtration et séchage, on a fait réagir 2,1 g du produit de la réaction précédente avec 2,62 g de triphényl phosphine pendant 15 heures à 130 °C sous atmosphère d'argon.

Le composé P23 de formule

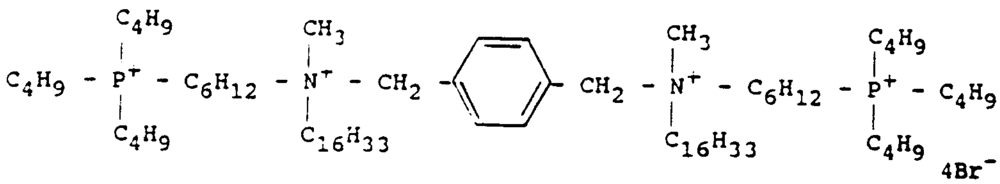

a été obtenu de la manière suivante :

On a fait réagir pendant 15 heures à 95 °C 27,83 g de méthyl hexadécyl amine avec 10,8 g de dibromo-p-xylène en milieu aqueux. On a récupéré un produit de réaction par extraction à l'éther isopropylique. Après lavage à l'eau et distillation du produit, on a fait réagir pendant 15 heures à 90 °C 3,83 g de ce produit avec 5,02 g du composé intermédiaire utilisé dans la préparation du composé P20.

Le composé P24 de formule :

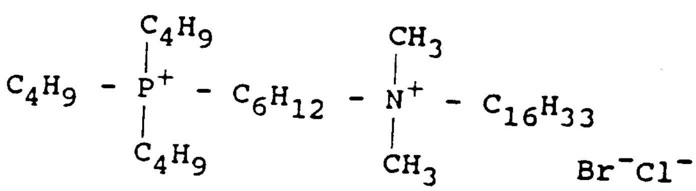

a été préparé en faisant réagir en milieu aqueux pendant 20 heures à 90 °C 4,04 g de diméthyl hexadécyl amine avec 6,03 g du composé intemédiaire utilisé dans la préparation du composé P20.

Le composé P25 de formule

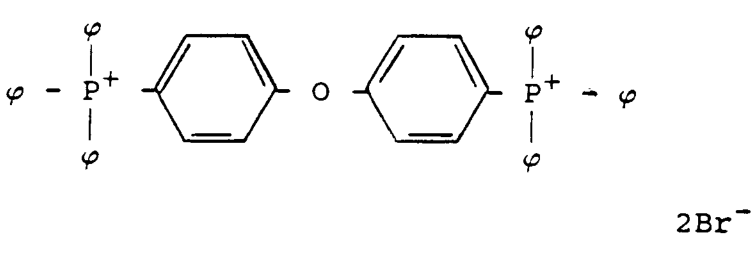

$\phi$ = phényle
a été préparé en faisant réagir sous atmosphère d'argon 8,2 g de 4-bromophényl éther et 13,11 g de triphényl phosphine pendant 20 heures à une température de 130 ° C.

Le composé P29 de formule :

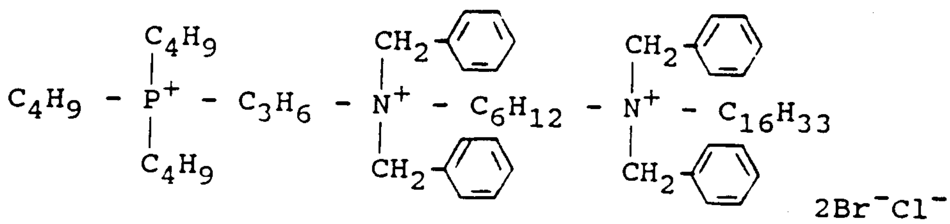

a été préparé en faisant réagir 2,7 g de bromure de tributyl-3-chloropropyl phosphonium avec 3,57 g de N,N,N',N'-tétrabenzyl hexane diamine en milieu aqueux pendant 20 heures à une température de 70 ° C. On a ensuite ajouté audit mélange 2,29 g de 1-bromohexadécane et on a maintenu la température du mélange à 90 ° C pendant 24 heures.

De manière similaire, on a préparé les composés suivants :

(P18)

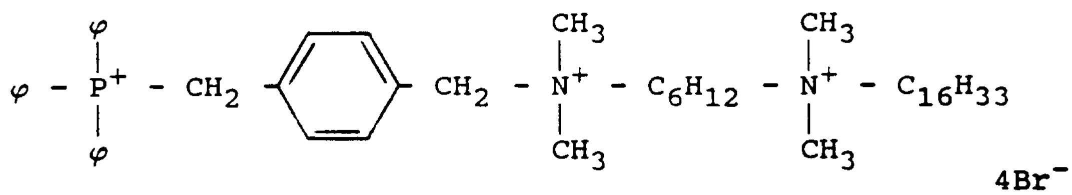

$\phi$ = phényle

(P19)

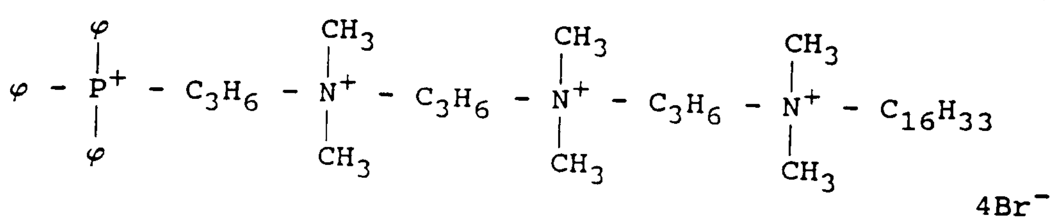

$\phi$ = phényle

(P26)

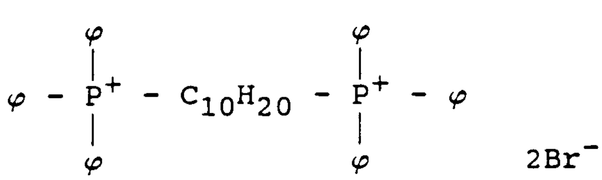

ϕ = phényle

(P30)

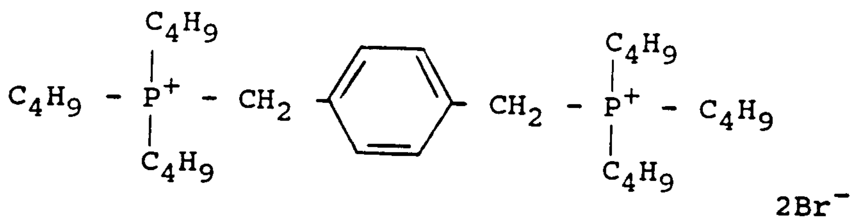

(P31)

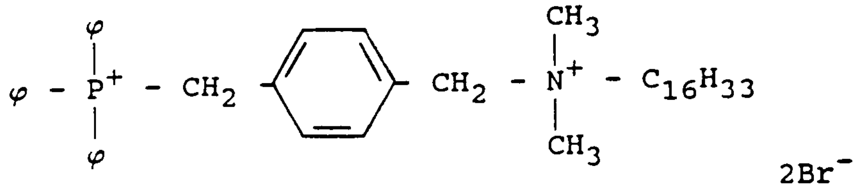

ϕ = phényle

(P33)

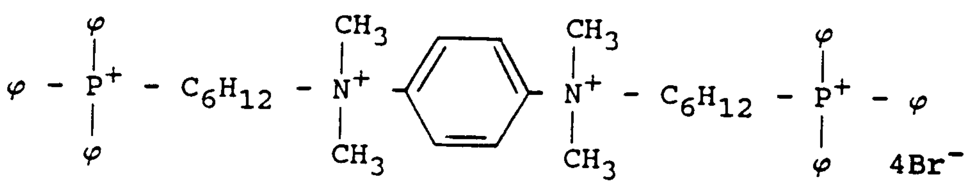

ϕ = phényle

On a préparé un composé P35 de formule

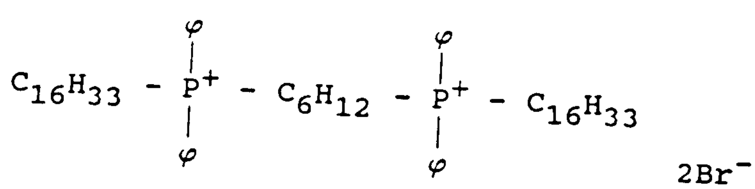

ϕ = phényle

(poids moléculaire moyen 1065 g) en mélangeant 0,909 g de 1,6-bis-(diphényl phosphine) hexane avec 1,22 g de 1-bromohexadécane et en portant ce mélange à 140 ° C à reflux. Après 4 heures, on a purifié le composé par cristallisation au moyen d'éther diéthylique anhydryde.

Préparation du composé phosphonium P36 :

On a mélangé dans 5 ml de $CH_2Cl_2$ 1,81 g de 1,6-bis-(diphénylphosphine)-hexane et 0,6 g de 1,10-dibromodécane. Le rapport molaire phosphine/dibromure du mélange était donc d'environ 2. Après avoir agité le mélange pendant 5 minutes, on a évaporé le solvant et on a porté le mélange à 135 ° C sous atmosphère d'argon et sous agitation. On a arrêté la réaction après 3 heures et après refroidissement, on a resolubilisé dans un peu de $CH_2Cl_2$ le produit de réaction. On a ensuite ajouté à la solution de l'éther

anhydre de manière à précipiter un produit qui, par après, a été filtré et séché sous vide.

On a ainsi obtenu avec un bon rendement un composé intermédiaire de formule

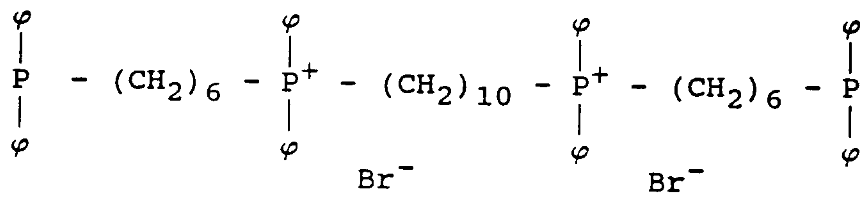

$\phi$ = phényle

On a ensuite mélangé 1 mole du produit intermédiaire avec 2 moles de bromohexadécane à 145 °C sous atmosphère d'argon pendant 4 heures. Après avoir refroidi le mélange, on a solubilisé ledit mélange dans du $CH_2Cl_2$ et on a ajouté de l'éther anhydre pour cristalliser un produit que l'on a récupéré par filtration et que l'on a séché sous vide.

On a ainsi obtenu le produit P36 de formule

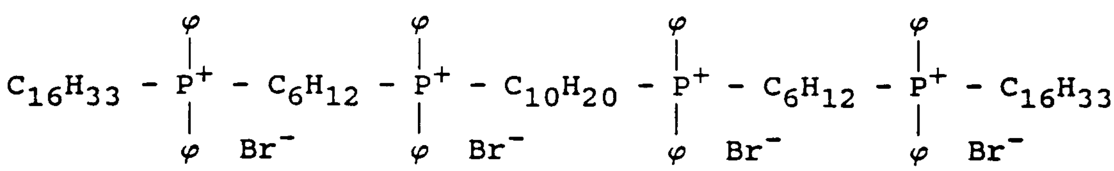

$\phi$ = phényle

Des composés d'ammonium qui peuvent être utilisés dans des compositions suivant l'invention peuvent être préparés par les méthodes décrites dans les documents US-A-4 217 914, GB-2 160 538, WO-A-87 02221 et WO-A-90 09405.

A titre d'exemples uniquement, des composés d'ammonium qui peuvent être utilisés dans des compositions suivant l'invention et qui ont un poids moléculaire supérieur à 1000 sont les composés de formule suivante :

(A1)

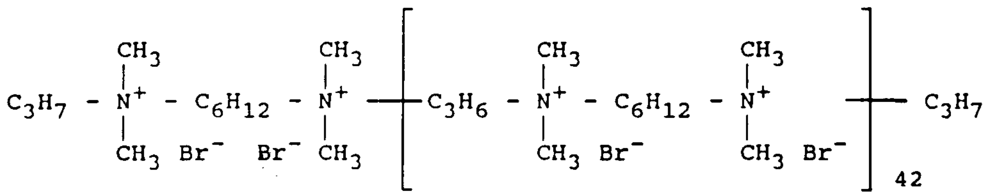

(A2)

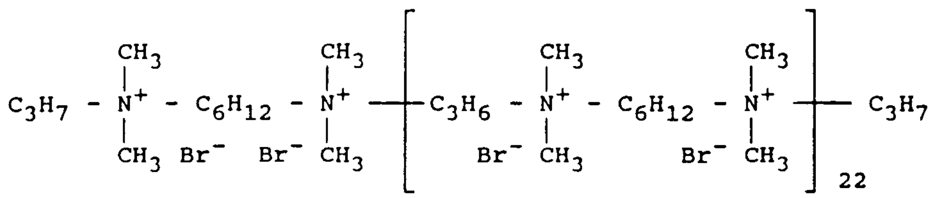

(A6)

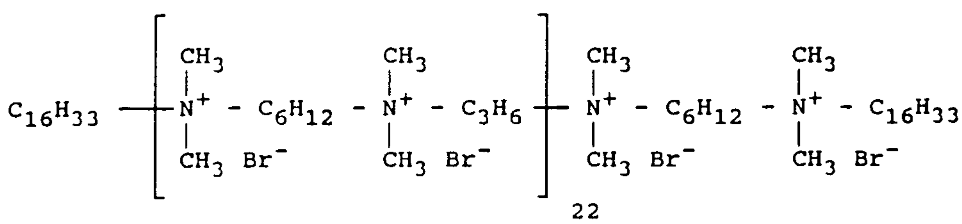

(A9)

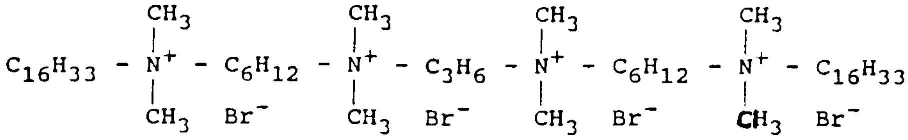

Le composé d'ammonium A8 utilisé dans les exemples d'utilisation 1 à 4 a été préparé de la manière suivante :

On a mélangé 5,17 g de tétraméthylhexane diamine avec 3,03 g de 1,3-dibromopropane dans 32,9 ml d'eau.

On a porté le mélange à 60 ° C et on a agité ledit mélange pendant 4 heures.

On a ensuite porté le mélange à 85 ° C et on a ajouté 9,16 g de bromohexadécane.

On a agité la solution pendant 12 heures.

On a ensuite dilué la solution obtenue avec une quantité d'eau correspondant à 20 fois le poids de la solution et on a traité ladite solution au charbon actif.

Le composé d'ammonium avait un poids moléculaire moyen en poids de 1157 g.

A titre de comparaison, des compositions contenant un phosphonium et un ammonium ayant un poids moléculaire inférieur à 1000 ont été préparées et testées. Ces compositions contenaient un des composés d'ammonium suivants :

(A15)

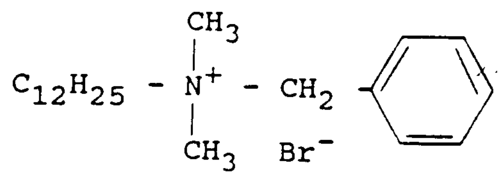

(A17)

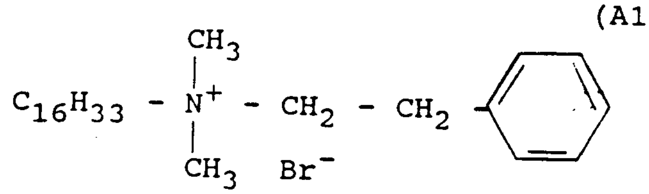

(A19)

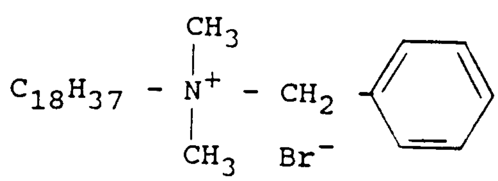

EXEMPLES D'UTILISATION

Pour effectuer ces essais, on a utilisé la méthode bien connue de dilutions successives dans des tubes à essais et ensemencement sur un milieu de culture tryptone-glucose-extract agar et en utilisant un polyphosphate comme neutralisant.

Les essais de désinfection d'eau dont il est question ci-dessous ont été effectués dans de l'eau de ville ayant une dureté d'environ 35°f, préalablement filtrée sur un filtre millipore de 0,45 $\mu$, après trois repiquages successifs de chacune des souches dans un milieu. Pour la culture des germes Streptococcus faecalis et Pseudomonas stutzeri, on a utilisé comme milieu de culture respectivement un milieu Slanetz et une solution aqueuse de 250 ml contenant 1 g/l de tryptone et 8,5 g de NaCl, tandis que pour la culture de tous les autres germes, on a utilisé un milieu gélose nutrive (OXOID CM 3).

Dans le procédé suivant l'invention pour la désinfection d'un milieu aqueux, on utilise, de préférence, au moins un composé de phosphonium à raison de 0,1 à 5 parties par million de parties du milieu aqueux à désinfecter et de 0,5 à 100 ppm d'au moins un composé d'ammonium de poids moléculaire supérieur à 1000. Quant aux ions de métaux, qui peuvent être produits au sein du milieu aqueux par électrolyse ou par addition à celui-ci de sels hydrosolubles de ces métaux, tels que sulfate, chlorure, nitrate, ils sont utilisés à

des concentrations par exemple de 0,1 à 1,5 ppm pour les ions de cuivre et/ou de 1 à 50 ppb, plus particulièrement de 1 à 10 ppb, pour les ions d'argent.

Pour le procédé décrit ci-dessus, des compositions pour le traitement d'eaux en vue de leur désinfection peuvent se présenter sous forme d'un mélange solide ou d'une solution ou dispersion aqueuse contenant au moins un composé hydrosoluble d'un métal choisi parmi le cuivre, l'argent, le manganèse et le zinc, au moins un composé de phosphonium quaternaire et au moins un composé d'ammonium de poids moléculaire supérieur à 1000.

L'action synergique inattendue du procédé et de la composition décrits ci-dessus est illustrée par les essais comparatifs suivants, basés sur la vitesse de destruction de divers micro-organismes choisis à titre d'exemples non limitatifs parmi les micro-organismes infestant couramment les eaux de piscines de natation.

Les mesures des vitesses de destruction des germes de micro-organismes ont été effectuées, dans les exemples illustratifs suivants, selon la méthode AOAC (American Official Analytical Chemist, 1980, pages 58 à 68) modifiée comme suit : utilisation de produit neutralisant dans le premier tube de dilution de l'échantillon de suspension bactérienne, dilutions de l'échantillon effectuées selon des facteurs de 10 en 10 jusqu'au facteur 100.000 immédiatement après la prise d'échantillons, incorporation directe dans le milieu de culture d'une quantité de l'échantillon non dilué.

Par germes stressés dans les exemples d'utilisation suivants, on entend des germes qui, avant leur utilisation, n'ont pas été repiqués dans le milieu de culture à trois reprises toutes les 24 heures. De tels germes présentent une résistance accrue aux agents bactéricides.

Exemple 1

| | |
|---|---|
| Souche bactérienne : | Staphylococcus aureus ATCC 6538 |
| Milieu aqueux : | eau de ville (dureté : 35 ° F*) filtrée sur filtre Millipore 0,45 µ |
| Concentration : | $1.10^6$ germes par ml |
| Composé de phosphonium quaternaire utilisé : | bromure de triphényl hexadécyl phosphonium P2 |

Le composé d'ammonium quaternaire utilisé avait la formule A8.

| Vitesse de destruction des germes bactériens | |
|---|---|
| Quantité et nature des ingrédients ajoutés | % de bactéries restantes après 30 minutes de contact |
| - 1 ppm ions cuivre | 95 |
| - 2 ppm de composé d'ammonium + 1 ppm ions cuivre | 0,0001 |
| - 1,9 ppm de composé d'ammonium + 0,1 ppm de composé de phosphonium + 1 ppm ions cuivre | 0,00000 |
| - 0,1 ppm de composé de phosphonium + 1 ppm ions cuivre | 0,12 |

Exemple 2

| | |
|---|---|
| Souche bactérienne : | Escherichia coli ATCC 11229 |
| Milieu aqueux : | eau de ville (dureté : 35 ° F) filtrée sur filtre Millipore 0,45 µ |
| Concentration initiale en germes : | $0,95.10^6$ germes par ml |
| Composé de phosphonium quaternaire utilisé : | bromure de triphényl hexadécyl phosphonium P2 |
| Composé d'ammonium quaternaire utilisé : | de formule A8 |

* °F = ° français

| Vitesse de destruction des germes bactériens | |
|---|---|
| Quantité et nature des ingrédients ajoutés | % de bactéries restantes après 30 minutes de contact |
| - 1 ppm ions cuivre + 2 ppm de composé d'ammonium | 0,04 |
| - 1 ppm ions cuivre + 1,9 ppm de composé d'ammonium + 0,1 ppm de composé de phosphonium | 0,004 |
| - 1 ppm ions cuivre + 1,8 ppm de composé d'ammonium + 0,2 ppm de composé de phosphonium | 0,00002 |

Exemple 3

Souche bactérienne : Pseudomonas aeruginosa ATCC 17939
Milieu aqueux : eau de ville (dureté : 35 ° F) filtrée sur filtre Millipore 0,45 µ
Concentration initiale en germes : $1,3.10^6$ germes par ml
Composé de phosphonium quaternaire utilisé : bromure de triphényl hexadécyl phosphonium P2
Composé d'ammonium quaternaire utilise : de formule A8

| Vitesse de destruction des germes bactériens | |
|---|---|
| Quantité et nature des ingrédients ajoutés | % de bactéries restantes après 30 minutes de contact |
| - 1 ppm ions cuivre + 2 ppm de composé d'ammonium | 0,005 |
| - 1 ppm ions cuivre + 1,9 ppm de composé d'ammonium + 0,1 ppm de composé de phosphonium | 0,0008 |
| - 1 ppm ions cuivre + 1,8 ppm de composé d'ammonium + 0,2 ppm de composé de phosphonium | 0,00005 |

Exemple 4

Souche bactérienne : Acinetobacter lwoffi
Milieu aqueux : eau de ville (dureté : 35 ° F) filtrée sur filtre Millipore 0,45 µ
Concentration initiale en germes : $1.10^6$ germes par ml
Composé de phosphonium quaternaire utilisé : bromure de triphényl hexadécyl phosphonium P2
Composé d'ammonium quaternaire utilisé : de formule A8

| Vitesse de destruction des germes bactériens | |
|---|---|
| Quantité et nature des ingrédients ajoutés | % de bactéries restantes après 30 minutes de contact |
| - 1 ppm ions cuivre + 2 ppm de composé d'ammonium | 0,03 |
| - 1 ppm ions cuivre + 1,9 ppm de composé d'ammonium + 0,1 ppm de composé de phosphonium | 0,005 |
| - 1 ppm ions cuivre + 1,8 ppm de composé d'ammonium + 0,2 ppm de composé de phosphonium | 0,0002 |

Exemple 5

Souche bactérienne : Pseudomonas stutzeri (souche isolée d'une piscine)
Milieu aqueux : eau de ville (dureté : 35 ° F) filtrée sur filtre Millipore 0,45 µ

Concentration initiale en germes : 90.10⁵ germes par ml

| Vitesse de destruction des germes bactériens | | |
|---|---|---|
| Quantité et nature des ingrédients ajoutés | % de bactéries restantes après | |
| | temps de contact | % |
| 1,9 ppm de composé ammonium A9 + 0,1 ppm de composé phosphonium P2 | 90 minutes<br>24 heures | 8<br>0,00003 |
| exemple comparatif 1,9 ppm de composé ammonium A16 + 0,1 ppm de composé phosphonium P2 | 90 minutes<br>24 heures | 41<br>0,00013 |

Exemple 6

Souche bactérienne : Proteus vulgaris (ATCC 13315)
Milieu aqueux : eau de ville (dureté : 35 °F) filtrée sur filtre Millipore 0,45 μ
Concentration initiale en germes : 3.000.000 germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| 2 ppm de composé ammonium A8 + 1 ppm Cu | 10<br>15 | 0,02<br>0,0003 |
| 1,8 ppm de composé ammonium A8 + 0,2 ppm de composé phosphonium P2 + 1 ppm Cu | 10<br>15 | 0,0004<br>0,00004 |

Exemple comparatif 7

Souche bactérienne : Proteus vulgaris (ATCC 13315)
Milieu aqueux : eau de ville (dureté : 35 °F) filtrée sur filtre Millipore 0,45 μ
Concentration initiale en germes : 3.000.000 germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| 2 ppm de composé ammonium A15 + 1 ppm Cu | 10<br>15 | 0,3<br>0,09 |
| 1,8 ppm de composé ammonium A15 + 0,2 ppm de composé phosphonium P2 + 1 ppm Cu | 10<br>15 | 6,2<br>2,2 |

La comparaison des exemples 6 et 7 montre clairement que l'utilisation combinée d'un phosphonium et d'un ammonium de poids moléculaire supérieur à 1000 (1156) permet une destruction beaucoup plus

rapide que celle obtenue en utilisant le même composé de phosphonium et un ammonium de poids moléculaire inférieur à 1000 (385). Seul un effet de synergie dû à la présence du composé phosphonium existait pour la destruction de la souche Proteus vulgaris lorsqu'on utilisait un composé d'ammonium de poids moléculaire supérieur à 1000.

Exemple 8

| | |
|---|---|
| Souche bactérienne : | Proteus vulgaris (ATCC 13315) |
| Milieu aqueux : | eau de ville (dureté : 35 ° F) filtrée sur filtre Millipore 0,45 µ |
| Concentration initiale en germes : | 700.000 germes par ml |

| Vitesse de destruction des germes bactériens | | |
|---|---|---|
| Quantité et nature des ingrédients ajoutés | % de bactéries restantes après un temps de contact de x minutes | |
| | x minutes | % |
| suivant l'invention : 1,8 ppm du composé ammonium A9 + 0,2 ppm du composé phosphonium P3 + 1 ppm Cu | 5 | 0,0003 |
| Exemple comparatif : 1,8 ppm du composé ammonium A15 + 0,2 ppm du composé phosphonium P3 + 1 ppm Cu | 5 | 0,13 |
| 1,8 ppm du composé ammonium A17 + 0,2 ppm du composé phosphonium P3 + 1 ppm Cu | 5 | 0,1 |

Exemple 9

Souche bactérienne : Pseudomonas stutzeri (germe isolé d'une piscine et identifié par le Laboratoire SIMON de Bierges, Belgique).

| | |
|---|---|
| Milieu aqueux : | eau de ville (dureté : 35 ° F) filtrée sur filtre Millipore 0,45 µ |
| Concentration initiale en germes : | 5.000.000 germes par ml |

| Vitesse de destruction des germes bactériens | | |
|---|---|---|
| Quantité et nature des ingrédients ajoutés | % de bactéries restantes après un temps de contact de x minutes | |
| | x minutes | % |
| 2 ppm de composé ammonium A9 + 1 ppm Cu | 30 | 18 |
| 1,8 ppm de composé ammonium A9 + 0,2 ppm de composé phosphonium P3 + 1 ppm Cu | 30 | 0,48 |
| 2 ppm de composé ammonium A15 + 1 ppm Cu | 30 | 74 |
| 1,8 ppm de composé ammonium A15 + 0,2 ppm de composé phosphonium P3 + 1 ppm Cu | 30 | 64 |

Ce tableau montre clairement l'effet de synergie qui existe pour la destruction de Pseudomonas stutzeri lorsqu'on a utilisé un composé de phosphonium et un composé ammonium de poids moléculaire supérieur à 1000, et qu'un tel effet de synergie n'existe pas lorsque le poids moléculaire du composé ammonium (A15) est inférieur à 1000.

Exemple 10

| | |
|---|---|
| Souche bactérienne : | Pseudomonas aeruginosa (ATCC 10145) |
| Milieu aqueux : | eau de ville (dureté : 35 ° F) filtrée sur filtre Millipore 0,45 µ |
| Concentration initiale en germes : | $64.10^5$ germes par ml |

| Vitesse de destruction des germes bactériens | | |
|---|---|---|
| Quantité et nature des ingrédients ajoutés | % de bactéries restantes après un temps de contact de x minutes | |
| | x minutes | % |
| 2 ppm composé ammonium A9 | 30 | 0,88 |
| 1,8 ppm composé ammonium A9 + 0,2 ppm composé phosphonium P2 | 30 | 0,078 |
| 2 ppm composé ammonium A9 + 1 ppm Cu | 30 | 0,018 |
| 1,8 ppm composé ammonium A9 + 0,2 ppm composé phosphonium P2 + 1 ppm Cu | 30 | 0,001 |
| 2 ppm composé ammonium A16 | 30 | 15 |
| 1,8 ppm composé ammonium A16 + 0,2 ppm composé phosphonium P2 | 30 | 15 |

Ce tableau montre d'une part l'effet de synergie lors de l'utilisation simultanée d'un composé d'ammonium d'un poids moléculaire supérieur à 1000 et d'un composé phosphonium et que cet effet de synergie est encore amplifié grâce à l'utilisation d'un ion d'un métal, à savoir le cuivre.

Exemple 11

Souche bactérienne : Klebsiella pneumoniae (ATCC 13833)
Milieu aqueux : eau de ville (dureté : 35 ° F) filtrée sur filtre Millipore 0,45 µ
Concentration initiale en germes : $24.10^5$ germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| - 2 ppm de composé phosphonium P3 | 5 | 82 |
| - 2 ppm de composé ammonium A6 | 5 | 44 |
| - 1,8 ppm de composé ammonium A6 + 0,2 ppm de composé phosphonium P3 | 5 | 17 |
| - 2 ppm de composé ammonium A15 | 5 | 90 |
| - 2 ppm de composé ammonium A16 | 5 | 99 |
| - 1,8 ppm de composé ammonium A15 + 0,2 ppm de composé phosphonium P3 | 5 | 90 |
| - 1,8 ppm de composé ammonium A16 + 0,2 ppm de composé phosphonium P3 | 5 | 82 |

Dans cet exemple, la bactérie a été stressée, puisqu'avant son emploi, elle n'a été repiquée qu'une seule fois.

Les résultats de cet exemple montrent clairement que seule une destruction rapide des germes de cette bactérie stressée peut être obtenue par l'utilisation simultanée d'un composé phosphonium et d'un composé d'ammonium ayant un poids moléculaire supérieur à 1000.

Exemple 12

Souche bactérienne : Escherichia coli (ATCC 11229)
Milieu aqueux : eau de ville (dureté : 35 ° F) filtrée sur filtre Millipore 0,45 µ
Concentration initiale en germes : $35.10^5$ germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| - 2 ppm de composé ammonium A9 + 1 ppm Cu | 5 | 0,1 |
| - 2 ppm de composé phosphonium P30 + 1 ppm Cu | 5 | 100 |
| - 2 ppm de composé phosphonium P3 + 1 ppm Cu | 5 | 89 |
| - 1,8 ppm de composé ammonium A9 + 0,2 ppm de composé phosphonium P30 + 1 ppm Cu | 5 | 0,003 |
| - 1,8 ppm de composé ammonium A9 + 0,2 ppm de composé phosphonium P3 + 1 ppm Cu | 5 | 0,01 |

| Vitesse de destruction des germes bactériens | | |
|---|---|---|
| Quantité et nature des ingrédients ajoutés | % de bactéries restantes après un temps de contact de x minutes | |
| | x minutes | % |
| - 1,8 ppm de composé ammonium A9 + 0,2 ppm de composé phosphonium P17 + 1 ppm Cu | 5 | 0,02 |
| - 1,8 ppm de composé ammonium A9 + 0,2 ppm de composé phosphonium P28 + 1 ppm Cu | 5 | 0,01 |
| - 1,8 ppm de composé ammonium A15 (poids moléculaire < 1000) + 0,2 ppm de composé phosphonium P3 + 1 ppm Cu | 5 | 47 |
| - 1,8 ppm de composé ammonium A16 (poids moléculaire < 1000) + 0,2 ppm de composé phosphonium P3 + 1 ppm Cu | 5 | 12 |

Ce tableau montre que les composés phosphonium P3 et P30 n'ont aucune efficacité pour détruire, en combinaison avec des ions Cu, des germes d'Escherichia coli, mais qu'ils permettent, lorsqu'ils sont utilisés dans des compositions contenant au moins un composé ammonium de poids moléculaire supérieur à 1000, d'accroître l'efficacité dudit composé ammonium.

Exemple 13

| | |
|---|---|
| Souche bactérienne : | Escherichia coli (ATCC 11229) |
| Milieu aqueux : | eau de ville (dureté : 35 ° F) filtrée sur filtre Millipore 0,45 µ |
| Concentration initiale en germes : | $35.10^5$ germes par ml |

| Vitesse de destruction des germes bactériens | | |
|---|---|---|
| Quantité et nature des ingrédients ajoutés | % de bactéries restantes après un temps de contact de x minutes | |
| | x minutes | % |
| - 3 ppm de composé ammonium A1 + 1 ppm Cu | 5 | 0,35 |
| - 3 ppm de composé ammonium A2 + 1 ppm Cu | 5 | 0,12 |
| - 2 ppm de composé phosphonium P3 + 1 ppm Cu | 5 | 89 |
| - 2,7 ppm de composé ammonium A1 + 0,3 ppm de composé phosphonium P3 + 1 ppm Cu | 5 | 0,029 |
| - 2,7 ppm de composé ammonium A2 + 0,3 ppm de composé phosphonium P3 + 1 ppm Cu | 5 | 0,0025 |
| - 2,7 ppm de composé ammonium A2 + 0,3 ppm de composé phosphonium P2 + 1 ppm Cu | 5 | 0,005 |

Ce tableau montre que l'action des compositions contenant un composé ammonium de poids moléculaire supérieur à 1000 pour détruire les germes d'Escherichia coli peut être améliorée en ajoutant auxdites compositions un composé phosphonium, composé qui semble être inactif en tant que tel.

Exemple 14

| | |
|---|---|
| Souche bactérienne : | Salmonella (LMG 326) (LMG Culture Collection, Laboratorium voor Microbiologie, Rijksuniversiteit Gent, K.L. Ledeganckstraat 35, B-9000 GENT, Belgique) |
| Milieu aqueux : | eau de ville (dureté : 35°F) filtrée sur filtre Millipore 0,45 µ |
| Concentration initiale en germes : | $18.10^5$ germes par ml |

| Vitesse de destruction des germes bactériens | | |
|---|---|---|
| Quantité et nature des ingrédients ajoutés | % de bactéries restantes après un temps de contact de x minutes | |
| | x minutes | % |
| - 1,8 ppm de composé ammonium A9 + 0,2 ppm de composé phosphonium P2 | 20 | 0,4 |
| - 1,8 ppm de composé ammonium A9 + 0,2 ppm de composé phosphonium P3 | 20 | 0,1 |
| - 1,5 ppm de composé ammonium A9 + 0,5 ppm de composé phosphonium P3 | 30 | 0,85 |
| - 1,8 ppm de composé ammonium A9 + 0,2 ppm de composé phosphonium P30 | 20 | 0,22 |

| Vitesse de destruction des germes bactériens | | |
|---|---|---|
| Quantité et nature des ingrédients ajoutés | % de bactéries restantes après un temps de contact de x minutes | |
| | x minutes | % |
| - 1,8 ppm de composé ammonium A9 + 0,2 ppm de composé phosphonium P24 | 20 | 0,4 |
| - 2,25 ppm de composé ammonium A2 + 0,75 ppm de composé phosphonium P3 | 30 | 1,02 |
| - 1,5 ppm de composé ammonium A7 + 0,5 ppm de composé phosphonium P3 | 30 | 0,58 |
| - 1,5 ppm de composé ammonium A16 (poids moléculaire < 1000) + 0,5 ppm de composé phosphonium P3 | 30 | 18,75 |
| - 1,8 ppm de composé ammonium A16 + 0,2 ppm de composé phosphonium P3 | 20 | 12 |
| - 1,8 ppm de composé ammonium A15 + 0,2 ppm de composé phosphonium P3 | 20 | 79 |

Exemple 15

| | |
|---|---|
| Souche bactérienne : | Escherichia coli (ATCC 11229) |
| Milieu aqueux : | eau de ville (dureté : 35 ° F) filtrée sur filtre Millipore 0,45 µ |
| Concentration initiale en germes : | $115.10^4$ germes par ml |

| Vitesse de destruction des germes bactériens | | |
|---|---|---|
| Quantité et nature des ingrédients ajoutés | % de bactéries restantes après un temps de contact de x minutes | |
| | x minutes | % |
| - 2 ppm de composé phosphonium P3 + 1 ppm Cu | 10 | 0,7 |
| - 2 ppm de composé ammonium A9 + 1 ppm Cu | 10 | 0,7 |
| - 1,8 ppm de composé ammonium A9 + 0,2 ppm de composé phosphonium P3 + 1 ppm Cu | 10 | 0,05 |
| - 0,5 ppm de composé ammonium A9 + 1,5 ppm de composé phosphonium P3 + 1 ppm Cu | 10 | 0,04 |
| - 2 ppm de composé ammonium A16 (poids moléculaire < 1000) + 1 ppm Cu | 10 | 30 |

| Vitesse de destruction des germes bactériens | | |
|---|---|---|
| Quantité et nature des ingrédients ajoutés | % de bactéries restantes après un temps de contact de x minutes | |
| | x minutes | % |
| - 1,8 ppm de composé ammonium A16 + 0,2 ppm de composé phosphonium P2 + 1 ppm Cu | 10 | 42 |
| - 0,5 ppm de composé ammonium A16 + 1,5 ppm de composé phosphonium P2 + 1 ppm Cu | 10 | 18 |

Cet exemple montre que seul un effet de synergie est observé lorsqu'on utilise une composition contenant un composé phosphonium et un composé ammonium de poids moléculaire supérieur à 1000.

Exemple 16

Souche bactérienne : Escherichia coli stressée (deux repiquages de la souche avant son utilisation)
Milieu aqueux : eau de ville (dureté : 35 ° F) filtrée sur filtre Millipore 0,45 µ
Concentration initiale en germes : $18.10^5$ germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| - 2 ppm de composé ammonium A7 + 1 ppm Cu | 15 | 52 |
| - 2 ppm de composé phosphonium P29 + 1 ppm Cu | 15 | 100 |
| - 1,8 ppm de composé ammonium A7 + 0,2 ppm de composé phosphonium P29 + 1 ppm Cu | 15 | 2,07 |
| - 0,5 ppm de composé ammonium A7 + 1,5 ppm de composé phosphonium P29 + 1 ppm Cu | 15 | 5,25 |

Exemple 17

Souche bactérienne : Staphilococcus hominis (souche isolée d'une eau d'une piscine et identifiée par le Laboratoire SIMON, 10 vieux chemin du Poète, Bierges, Belgique)
Milieu aqueux : eau de ville (dureté : 35 ° F) filtrée sur filtre Millipore 0,45 µ
Concentration initiale en germes : $24.10^5$ germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| - 2 ppm de composé ammonium A9 | 5 | 0,0067 |
| - 1,8 ppm de composé ammonium A9 + 0,2 ppm de composé phosphonium P32 | 5 | 0,00067 |
| - 3 ppm de composé ammonium A2 | 5 | 0,039 |
| - 2,7 ppm de composé ammonium A2 + 0,3 ppm de composé phosphonium P3 | 5 | 0,001 |
| - 1,8 ppm de composé ammonium A15 + 0,2 ppm de composé phosphonium P3 | 5 | 0,02 |
| - 1,8 ppm de composé ammonium A16 + 0,2 ppm de composé phosphonium P3 | 5 | 3,9 |

Cet exemple montre que seule une bonne destruction des souches est obtenue grâce à l'utilisation d'un composé d'ammonium de poids moléculaire supérieur à 1000 et d'un composé de phosphonium.

Exemple 18

Souche bactérienne : Staphilococcus epidermidis (souche isolée d'une eau de piscine et identifiée par le Laboratoire SIMON, 10 vieux chemin du Poète,

| | |
|---|---|
| | Bierges, Belgique) |
| Milieu aqueux : | eau de ville (dureté : 35 ° F) filtrée sur filtre Millipore 0,45 μ |
| Concentration initiale en germes : | $79.10^3$ germes par ml |

| Vitesse de destruction des germes bactériens | | |
|---|---|---|
| Quantité et nature des ingrédients ajoutés | % de bactéries restantes après un temps de contact de x minutes | |
| | x minutes | % |
| 3 ppm de composé ammonium A2 | 5 | 4 |
| 2,7 ppm de composé ammonium A2 + 0,3 ppm de composé phosphonium P3 | 5 | 0,3 |

Exemple 19

| | |
|---|---|
| Souche bactérienne : | Escherichia coli (ATCC 11229) (germes stressés - deux repiquages avant son utilisation) |
| Milieu aqueux : | eau de ville (dureté : 35 ° F) filtrée sur filtre Millipore 0,45 μ |
| Concentration initiale en germes : | $20.10^5$ germes par ml |

| Vitesse de destruction des germes bactériens | | |
|---|---|---|
| Quantité et nature des ingrédients ajoutés | % de bactéries restantes après un temps de contact de x minutes | |
| | x minutes | % |
| 2 ppm de composé ammonium A9 + 1 ppm Cu | 10 | 8,5 |
| 2 ppm de composé ammonium A6 + 1 ppm Cu | 10 | 5,4 |
| 2 ppm de composé phosphonium P3O + 1 ppm Cu | 10 | 63 |
| 1,8 ppm de composé ammonium A9 + 0,2 ppm de composé phosphonium P30 + 1 ppm Cu | 10 | 0,1 |

| Vitesse de destruction des germes bactériens | | |
|---|---|---|
| Quantité et nature des ingrédients ajoutés | % de bactéries restantes après un temps de contact de x minutes | |
| | x minutes | % |
| 1,8 ppm de composé ammonium A9 + 0,2 ppm de composé phosphonium P3 + 1 ppm Cu | 10 | 0,025 |
| 1,8 ppm de composé ammonium A6 + 0,2 ppm de composé phosphonium P3 + 1 ppm Cu | 10 | 0,1 |
| 1,8 ppm de composé ammonium A6 + 0,2 ppm de composé phosphonium P30 + 1 ppm Cu | 10 | 0,1 |

Cet exemple montre que le remplacement de 0,2 ppm d'un composé ammonium de poids moléculaire > 1000 par un composé phosphonium permet d'accroître l'efficacité de la composition d'un facteur supérieur à 80, alors que ledit composé phosphonium en combinaison avec du cuivre est inefficace.

Exemple 20

Souche bactérienne : Proteus vulgaris (ATCC 13315)
Milieu aqueux : eau de ville (dureté : 35 ° F) filtrée sur filtre Millipore 0,45 µ
Concentration initiale en germes : $110.10^4$ germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| 1,8 ppm de composé ammonium A9 + 0,2 ppm de composé phosphonium P3 | 1<br><br>5<br>10<br>15 | 32<br><br>0,1<br>0,004<br>0,00009 |
| 1,8 ppm de composé ammonium A15 (poids moléculaire < 1000) + 0,2 ppm de composé phosphonium P3 | 1<br><br>5<br>10<br>15 | 100<br><br>93<br>68<br>45 |

Ce tableau montre l'efficacité d'une composition suivant l'invention, c'est-à-dire l'importance d'utiliser un composé phosphonium en combinaison avec un composé d'ammonium de poids moléculaire supérieur à 1000.

Exemple 21

Souche bactérienne : Escherichia coli (ATCC 11229)
Milieu aqueux : eau de ville (dureté : 35 ° F) filtrée sur filtre Millipore 0,45 µ
Concentration initiale en germes : $27.10^5$ germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| 3 ppm de composé ammonium A2 + 1 ppm Cu | 15 | 32 |
| 3 ppm de composé phosphonium P3 + 1 ppm Cu | 15 | 98 |
| 3 ppm de composé phosphonium P30 + 1 ppm Cu | 15 | 59 |
| 2,7 ppm de composé ammonium A2 + 0,3 ppm de composé phosphonium P3 + 1 ppm Cu | 15 | 6 |

| Vitesse de destruction des germes bactériens | | |
|---|---|---|
| Quantité et nature des ingrédients ajoutés | % de bactéries restantes après un temps de contact de x minutes | |
| | x minutes | % |
| 2,3 ppm de composé ammonium A2 + 0,7 ppm de composé phosphonium P3 + 1 ppm Cu | 15 | 2 |
| 1,5 ppm de composé ammonium A2 + 1,5 ppm de composé phosphonium P3 + 1 ppm Cu | 15 | 0,08 |
| 0,7 ppm de composé ammonium A2 + 2,3 ppm de composé phosphonium P3 + 1 ppm Cu | 15 | 0,2 |
| 0,3 ppm de composé ammonium A2 + 2,7 ppm de composé phosphonium P3 + 1 ppm Cu | 15 | 0,2 |
| 2,7 ppm de composé ammonium A2 + 0,3 ppm de composé phosphonium P30 + 1 ppm Cu | 15 | 1,5 |

| Vitesse de destruction des germes bactériens | | |
|---|---|---|
| Quantité et nature des ingrédients ajoutés | % de bactéries restantes après un temps de contact de x minutes | |
| | x minutes | % |
| 1,5 ppm de composé ammonium A2 + 1,5 ppm de composé phosphonium P30 + 1 ppm Cu | 15 | 0,16 |
| 0,3 ppm de composé ammonium A2 + 2,7 ppm de composé phosphonium P30 + 1 ppm Cu | 15 | 0,18 |

Cet exemple montre que la quantité d'ammonium et la quantité de phosphonium présentes dans la composition suivant l'invention peuvent varier dans une large gamme.

Exemple 22

| | |
|---|---|
| Souche bactérienne : | Proteus vulgaris (ATCC 13315) (germe stressé - deux repiquages de la souche avant son utilisation) |
| Milieu aqueux : | eau de ville (dureté : 35 ° F) filtrée sur filtre Millipore 0,45 µ |
| Concentration initiale en germes : | $28.10^5$ germes par ml |

| Vitesse de destruction des germes bactériens | | |
|---|---|---|
| Quantité et nature des ingrédients ajoutés | % de bactéries restantes après un temps de contact de x minutes | |
| | x minutes | % |
| 2 ppm du composé ammonium A9 | 10 | 0,012 |
| 2 ppm du composé phosphonium P3 | 10 | 65 |
| 1 ppm du composé ammonium A9 + 1 ppm du composé phosphonium P3 | 10 | 0,001 |

Exemple 23

| | |
|---|---|
| Souche bactérienne : | Pseudomonas stutzeri (souche isolée d'une eau de piscine et identifiée par le Laboratoire SIMON, 10 vieux chemin du Poète, Bierges, Belgique) |

Milieu aqueux : eau de ville (dureté : 35 ° F) filtrée sur filtre Millipore 0,45 μ
Concentration initiale en germes : $26.10^5$ germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| - 1,8 ppm de composé ammonium A9 + 0,2 ppm de composé phosphonium P20 | 60 | 0,06 |
| - 1,8 ppm de composé ammonium A9 + 0,2 ppm de composé phosphonium P26 | 60 | 0,061 |
| - 1,8 ppm de composé ammonium A9 + 0,2 ppm de composé phosphonium P23 | 60 | 0,07 |
| - 1,8 ppm de composé ammonium A16 (poids moléculaire < 1000) + 0,2 ppm de composé composé phosphonium P3 | 60 | 1,8 |

Ce tableau montre que le composé phosphonium de la composition suivant l'invention peut être un polyphosphonium ou un composé phosphonium ammonium.

Exemple 24

Souche bactérienne : Escherichia coli (ATCC 11229)
Milieu aqueux : eau de ville (dureté : 35 ° F) filtrée sur filtre Millipore 0,45 μ
Concentration initiale en germes : $186.10^4$ germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| 1,8 ppm de composé ammonium A16 + 0,2 ppm de composé phosphonium P3 | 10 | 79 |
| 1,8 ppm de composé ammonium A9 | 10 | 2,2 |
| 0,2 ppm de composé phosphonium P3 | 10 | 81 |
| 1,8 ppm de composé ammonium A9 + 0,2 ppm de composé phosphonium P3 | 10 | 0,14 |

| Vitesse de destruction des germes bactériens | | |
|---|---|---|
| Quantité et nature des ingrédients ajoutés | % de bactéries restantes après un temps de contact de x minutes | |
| | x minutes | % |
| 1,8 ppm de composé ammonium A9 + 0,2 ppm de composé phosphonium P20 | 10 | 0,22 |
| 1,8 ppm de composé ammonium A9 + 0,2 ppm de composé phosphonium P23 | 10 | 0,14 |
| 1,8 ppm de composé ammonium A9 + 0,2 ppm de composé phosphonium P26 | 10 | 0,17 |
| 1,8 ppm de composé ammonium A9 + 0,2 ppm de composé phosphonium P21 | 10 | 0,28 |

L'action remarquable des compositions suivant l'invention par rapport à l'action d'un ammonium seul, d'un phosphonium seul ou de la combinaison ammonium de poids moléculaire inférieur à 1000 - phosphonium ressort clairement de ce tableau.

Exemple 25

Souche bactérienne : Pseudomonas stutzeri (souche isolée d'une eau de piscine et identifiée par le Laboratoire SIMON, 10 vieux chemin du Poète, Bierges, Belgique)
Milieu aqueux : eau de ville (dureté : 35 °F) filtrée sur filtre Millipore 0,45 µ
Concentration initiale en germes : $25.10^5$ germes par ml

| Vitesse de destruction des germes bactériens | | |
|---|---|---|
| Quantité et nature des ingrédients ajoutés | % de bactéries restantes après un temps de contact de x minutes | |
| | x minutes | % |
| 1,8 ppm de composé ammonium A16 ( poids moléculaire < 1000) | 60 | 4,2 |
| 1,8 ppm de composé ammonium A9 | 60 | 0,38 |
| 0,2 ppm de composé phosphonium P3 | 60 | 50 |
| 1,8 ppm de composé ammonium A16 + 0,2 ppm de composé phosphonium P3 | 60 | 3,3 |

| Vitesse de destruction des germes bactériens | | |
|---|---|---|
| Quantité et nature des ingrédients ajoutés | % de bactéries restantes après un temps de contact de x minutes | |
| | x minutes | % |
| 1,8 ppm de composé ammonium A9 + 0,2 ppm de composé phosphonium P3 | 60 | 0,079 |
| 1,8 ppm de composé ammonium A9 + 0,2 ppm de composé phosphonium P21 | 60 | 0,045 |
| 1,8 ppm de composé ammonium A9 + 0,2 ppm de composé phosphonium P33 | 60 | 0,031 |

Ce tableau montre que seul un effet de synergie est obtenu lorsqu'on utilise en combinaison un composé d'ammonium de poids moléculaire supérieur à 1000 et un composé de phosphonium.

Exemple 26

Souche bactérienne : Proteus vulgaris (ATCC 13315)*
Milieu aqueux : eau de ville (dureté : 35 °F) filtrée sur filtre Millipore 0,45 µ
Concentration initiale en germes : $1.10^6$ germes par ml

* (germes stressés, un seul repiquage)

| Vitesse de destruction des germes bactériens | | |
|---|---|---|
| Quantité et nature des ingrédients ajoutés | % de bactéries restantes après un temps de contact de x minutes | |
| | x minutes | % |
| 2 ppm du composé ammonium A9 | 1 | 70 |
| 1,8 ppm du composé ammonium A9 + 0,2 ppm du composé phosphonium P36 | 1 | 4 |

Exemple 27

On a préparé dans de l'eau distillée une première composition A contenant 3,6 % en poids de composé ammonium A9 et 0,4 % en poids de composé phosphonium P3, et une deuxième composition B contenant 3,6 % en poids de composé ammonium A16 (poids moléculaire 1000) et 0,4 % en poids de composé phosphonium P3.

On a ajouté la composition A dans une première piscine et la composition B dans une deuxième piscine adjacente à la première. Chaque piscine contenait 800 litres d'eau de ville de 35°F de dureté et était munie d'un système de filtration continue sur sable (temps de séjour de l'eau dans la piscine = 95 minutes ; 66 % de l'eau envoyée au filtre est de l'eau débordant de la piscine), d'un système de régulation du pH à 7,6 et d'un système de régulation de la température à 28°C.

Dans chacune des piscines, on a maintenu une concentration de 2 ppm de composition désinfectante dans l'eau.

Les piscines étaient placées l'une à côté de l'autre dans un même bâtiment (même environnement de contamination) et ont été contaminées au moyen d'un litre d'une même suspension contenant $27.10^7$ germes cibles et banaux par ml. Les germes banaux étaient des germes du type Pseudomonas, Acinetobacter et Acetobacter.

On a ainsi remarqué que les germes cibles et banaux étaient rapidement tués dans la piscine traitée par la composition A suivant l'invention, et que des germes banaux du genre Pseudomonas, Staphylococcus, .... contaminaient de plus en plus la piscine traitée au moyen de la composition B.

Le tableau suivant reprend les résultats du traitement.

| Temps après addition de la composition désinfectante | germes restant dans la piscine en germes/ml | |
|---|---|---|
| | Composition A | Composition B |
| 5 minutes | $27.10^4$ | $54.10^4$ |
| 300 minutes | 89 | $97.10^2$ |
| 24 heures | 2 | $18.10^4$ |

Des essais sur des souches de champignons que l'on retrouve aux abords des piscines telles que Trichophyton rubrum ou Chrysosporium keratinophilium ont montré l'action fongicide en 24 heures de compositions suivant l'invention sous forme de solution aqueuse contenant moins de 50 ppm d'ammonium et de phosphonium et en présence de 30 ppm de Cu et 100 ppb de Ag.

L'invention a donc aussi pour objet une composition fongicide à usage externe par exemple pour le traitement de champignons se développant sur le corps humain ou sur le corps d'un animal, cette composition contenant au moins un composé suivant l'invention et, de préférence, un agent favorisant la pénétration dudit composé. Un tel agent est par exemple de l'éthanol ou un autre alcool.

Dans le cas où la composition ou le procédé suivant l'invention sont appliqués à la désinfection d'eau, on utilise, de préférence, au moins une composition suivant l'invention, à raison de 0,5 à 1000 parties par million (ppm) du milieu aqueux à désinfecter. Quant aux ions des métaux, tels que le cuivre et l'argent, qui peuvent être produits au sein de ce milieu aqueux par électrolyse ou par addition à celui-ci de sels hydrosolubles de ces métaux, tels que sulfate, chlorure, nitrate, ils sont utilisés, de préférence, à des concentrations de 0,5 à 5 ppm pour les ions de cuivre et de 1 à 50 ppb (parties par milliard), plus particulièrement de 1 à 10 ppb pour les ions d'argent.

Lorsqu'on doit désinfecter des surfaces, la composition suivant l'invention est projetée ou pulvérisée sur lesdites surfaces.

Il va de soi que, pour désinfecter des surfaces, il est également possible de tremper les surfaces à désinfecter dans un bain.

La composition suivant l'invention est également utile pour la conservation d'aliments ou de boissons puisqu'elle permet une destruction rapide des germes au moyen de très faibles quantités et qu'elle reste efficace pendant une longue période de temps.

La composition suivant l'invention peut, vu la faible quantité nécessaire pour obtenir la destruction rapide des germes, être utilisée dans la conservation de boissons, d'aliments.

La composition suivant l'invention peut ainsi être ajoutée pendant l'étape de cuisson de confitures, peut être injectée dans des aliments. On peut également imprégner ou imbiber des aliments d'une composition suivant l'invention en trempant lesdits aliments dans un bain contenant ladite composition ou en pulvérisant sur lesdits aliments ladite composition.

De façon avantageuse, la composition suivant l'invention peut être utilisée pour le dégraissage et la stérilisation de la laine.

La composition suivant l'invention peut être utilisée dans des savons, dentifrices, shampooings, pansements médicaux, pour la conservation d'aiguilles de seringues, de verres de contact, pour la stérilisation d'enzymes extraits de bactéries sans risque de dénaturation, pour la protection du bois, pour la conservation d'hydrocarbures, pétrole, papiers, cotons, pour la destruction de bactéries sulfato-réductrices, pour des traitements agricoles, antifongiques.

**Revendications**

**1.** Composition destinée à la désinfection de liquides et/ou de surfaces ou à la conservation d'aliments ou boissons, cette composition contenant au moins un composé d'ammonium quaternaire ayant un poids moléculaire compris entre 1000 et 50.000, de formule

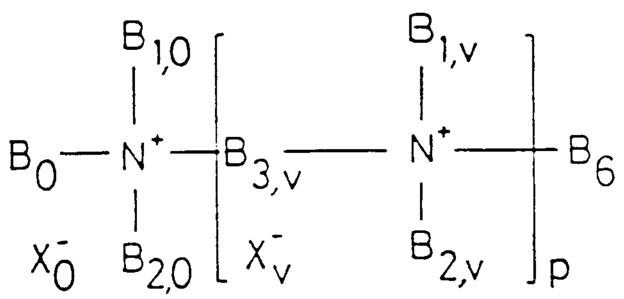

(I)

dans laquelle :

- p est un nombre entier au moins égal à 1 ;
- v est un nombre entier compris entre 1 et p ;
- $B_0$ et $B_6$ qui peuvent être identiques ou différents désignent un radical hydrocarboné éventuellement substitué et éventuellement insaturé, ce radical contenant de 1 à 22 atomes de carbone ;
- $B_{1,0}$ , $B_{2,0}$ , $B_{1,v}$ et $B_{2,v}$ pour v compris entre 1 et p désignent un groupe hydrocarboné éventuellement ramifié, insaturé et/ou substitué par un ou plusieurs halogènes ou par un groupement hydrocarboné ou par un groupement carboxyle ou par un groupement hydroxyle, $B_{1,0}$ , $B_{2,0}$ , $B_{1,v}$ et $B_{2,v}$ pouvant contenir jusqu'à 22 atomes de carbone, $B_{1,v}$ et/ou $B_{2,v}$ pouvant être reliés respectivement à $B_{1,v+1}$ et/ou $B_{1,v-1}$ et à $B_{2,v+1}$ et/ou $B_{2,v-1}$ ;
- $B_{3,v}$ pour v compris entre 1 et p désigne un groupe hydrocarboné éventuellement substitué insaturé et/ou ramifié pouvant contenir jusqu'à 20 atomes de carbone ou un groupe biguanide ou un groupe de formule

(1) o-, m- ou p-xylydène de formule :

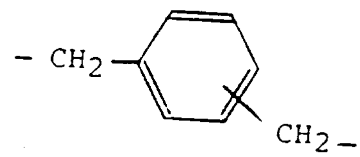

(2)

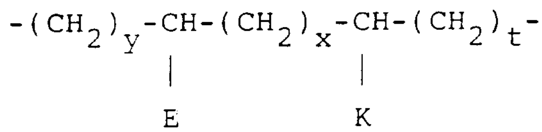

où x, y et t sont des nombres entiers allant de 0 à 11, tels que la somme de x + y + t est égale ou supérieure à 0 et inférieure à 18, tandis que les symboles E et K désignent l'hydrogène ou des radicaux alkyle contenant moins de 18 atomes de carbone ;

(3) $-(CH_2)_n-S-(CH_2)_n-$ ,

(4) $-(CH_2)_n-O-(CH_2)_n-$ ,

(5) $-(CH_2)_n-S-S-(CH_2)_n-$ ,

(6) $-(CH_2)_n-SO-(CH_2)_n-$ ,

(7) $-(CH_2)_n-SO_2-(CH_2)_n-$ ,

(8)

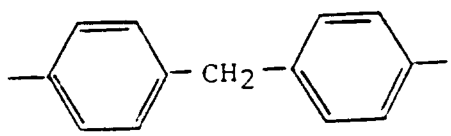

où n est égal à 1,2 ou 3.

(9)

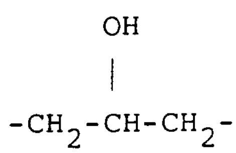

et

$X^-_o$ et $X^-_v$ désignent un anion, de préférence un atome d'halogène, et au moins un composé de formule

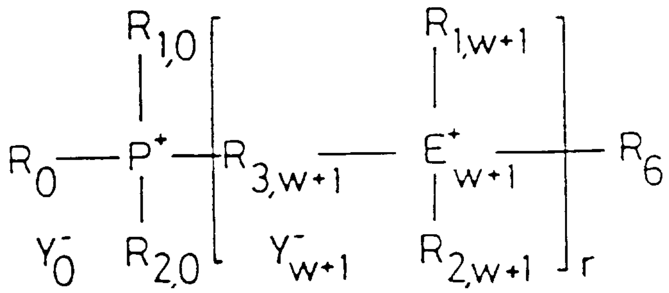

(II)

dans laquelle :

- r est un nombre entier éventuellement égal à 0 ;
- w est un nombre entier compris entre 0 et r ;
- $R_0$ et $R_6$ qui peuvent être identiques ou différents désignent un radical hydrocarboné éventuellement substitué et éventuellement insaturé, ce radical contenant de 1 à 22 atomes de carbone ;
- $R_{1,0}$ , $R_{2,0}$ , $R_{1,w+1}$ et $R_{2,w+1}$ pour w compris entre 0 et r désignent un groupe hydrocarboné éventuellement ramifié, insaturé et/ou substitué par un ou plusieurs halogènes ou par un groupement hydrocarboné ou par un groupement hydroxyle, $R_{1,w+1}$ et $R_{2,w+1}$ pouvant contenir jusqu'à 22 atomes de carbone, $R_{1,w+1}$ et/ou $R_{2,w+1}$ pouvant être reliés respectivement à $R_{1,w+2}$ et/ou $R_{1,w}$ et à $R_{2,w+2}$ et/ou $R_{2,w}$ ;
- $E^+_{w+1}$ étant choisi pour w compris entre 0 et r parmi N et P ;
- $R_{3,w+1}$ désigne un groupe hydrocarboné éventuellement insaturé et/ou ramifié pouvant contenir jusqu'à 20 atomes de carbone ou un groupe de formule :

(1) o-, m- ou p-xylylidène de formule :

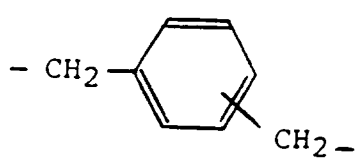

(2)

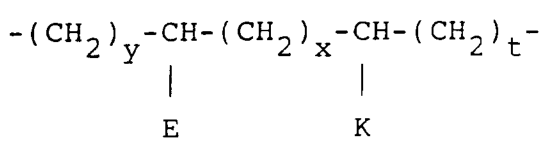

où x, y et t sont des nombres entiers allant de 0 à 11, tels que la somme de x + y + t est égale ou supérieure à 0 et inférieure à 18, tandis que les symboles E et K désignent de l'hydrogène ou des radicaux alkyle contenant moins de 18 atomes de carbone ;

(3) $-(CH_2)_n-S-(CH_2)_n-$ ,

(4) $-(CH_2)_n-O-(CH_2)_n-$ ,

(5) $-(CH_2)_n-S-S-(CH_2)_n-$ ,

(6) $-(CH_2)_n-SO-(CH_2)_n-$ ,

(7) $-(CH_2)_n-SO_2-(CH_2)_n-$ ,

(8)

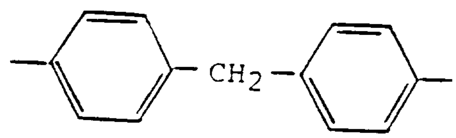

où n est égal à 1, 2 ou 3 ;
(9)

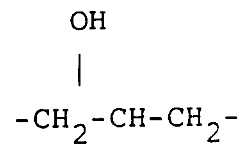

et
$Y^{-}_{0}$ et $Y^{-}_{w+1}$ désignent un anion, de préférence un halogène.

**2.** Composition suivant la revendication 1, caractérisée en ce que le composé d'ammonium quaternaire a un poids moléculaire compris entre 1000 et 5000.

**3.** Composition suivant l'une quelconque des revendications précédentes, caractérisée en ce qu'elle contient également au moins un ion d'un métal choisi parmi le fer, le cuivre, l'argent, l'or, le manganèse, le zinc ou un mélange de tels ions.

**4.** Composition suivant l'une quelconque des revendications précédentes, caractérisée en ce qu'elle contient de 5 à 95 % en poids de composé de formule II par rapport à la quantité totale de composé de formule I et de composé de formule II que contient la composition.

**5.** Composition suivant l'une quelconque des revendications précédentes, caractérisée en ce que l'anion est un atome de chlore ou de brome.

**6.** Composition suivant l'une quelconque des revendications précédentes, caractérisée en ce que $R_0$ et/ou $R_6$ et/ou $B_0$ et/ou $B_6$ contiennent de 12 à 22 atomes de carbone, de préférence 16 atomes de carbone.

**7.** Composition suivant l'une quelconque des revendications précédentes, caractérisée en ce que pour au moins un k compris entre 0 et r, $R_{1,k}$ et $R_{1,k+1}$ du composé de formule II sont reliés entre eux et/ou $R_{2,k}$ et $R_{2,k+1}$ du composé de formule II sont reliés entre eux en formant de préférence un groupe identique à $R_{3,k+1}$.

**8.** Composition suivant l'une quelconque des revendications précédentes, caractériséeen ce que pour au moins un l compris entre 0 et p, $B_{1,l}$ et $B_{1,l+1}$ du composé de formule I sont reliés entre eux et/ou $B_{2,l}$ et $B_{2,l+1}$ du composé de formule I sont reliés entre eux en formant de préférence un groupe identique à $B_{3,l+1}$.

**9.** Composition suivant la revendication 7 ou 8, caractérisée en ce que $R_{1,k}$ et $R_{1,k+1}$ et/ou $R_{2,k}$ et $R_{2,k+1}$ et/ou $B_{1,l}$ et $B_{1,l+1}$ et/ou $B_{2,l}$ et $B_{2,l+1}$ forment un groupe $-(CH_2)_2-$.

**10.** Composition suivant la revendication 1, caractérisée en ce que $R_{3,k+1}$ est un groupe de formule $-CH_2-CH_2-CH_2-$ lorsque k est un nombre pair, tandis que $R_{3,k+1}$ est un groupe de formule $-CH_2-CH_2-CH_2-CH_2-CH_2-CH_2-$lorsque k est un nombre impair, ou inversément.

**11.** Composition suivant la revendication 1, caractérisée en ce que $B_{3,l}$ est un groupe de formule $-CH_2-CH_2-CH_2-$ lorsque l est un nombre pair, tandis que $B_{3,l}$ est un groupe de formule $-CH_2-CH_2-CH_2-CH_2-$

$CH_2$-$CH_2$-lorsque l est un nombre impair, ou inversément.

**12.** Composition suivant la revendication 1, caractérisée en ce que $R_{3,k+1}$ et $B_{3,l}$ pour au moins un k ou l compris entre 0 et r ou 1 et p est un groupe de formule :

$$-(CH_2)_c-\underset{\underset{D}{|}}{CH}-(CH_2)_d-$$

dans laquelle D désigne un atome d'hydrogène ou un radical $C_{1-4}$ alkyle, c et d sont des nombres entiers inférieurs à 5 dont l'un peut avoir une valeur égale à 0 tandis que la somme c + d est au moins égale à 1 et au maximum égale à 8.

**13.** Procédé de désinfection de liquides, caractérisé en ce qu'on ajoute à ces liquides au moins une composition suivant l'une quelconque des revendications précédentes.

**14.** Procédé de désinfection de surfaces infectées, caractérisé en ce qu'on met ces surfaces en contact avec au moins une composition désinfectante suivant l'une quelconque des revendications 1 à 12.

**15.** Procédé de conservation de boissons dans lequel on ajoute auxdites boissons une composition suivant l'une quelconque des revendications 1 à 12.

**16.** Procédé de conservation d'aliments dans lequel on injecte dans lesdits aliments une composition suivant l'une quelconque des revendications 1 à 12.

**17.** Procédé de conservation d'aliments dans lequel on trempe lesdits aliments dans une composition suivant l'une quelconque des revendications 1 à 12.

## Claims

**1.** Composition intended for disinfecting liquids and/or surfaces or for preserving food or drinks, this composition containing at least one compound of quaternary ammonium having a molecular weight comprised between 1,000 and 50,000, of formula

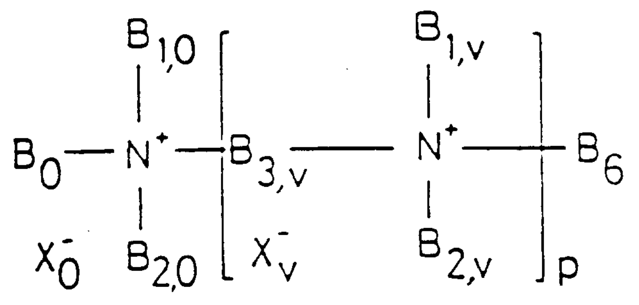

(I)

in which

- p is an integer at least equal to 1 ;
- v is an integer comprised between 1 and p ;
- $B_0$ and $B_6$ which may be identical or different refer to a hydrocarbon radical possibly substituted and possibly unsaturated, this radical containing from 1 to 22 carbon atoms ;
- $B_{1,0}$, $B_{2,0}$, $B_{1,v}$ and $B_{2,v}$, for v comprised between 1 and p, refer to a hydrocarbon group possibly ramified, unsaturated and/or substituted by one or more halogen atoms or by a hydrocarbon group or by a carboxyl group or by a hydroxyl group, whereby $B_{1,0}$, $B_{2,0}$, $B_{1,v}$ and $B_{2,v}$ may contain up to 22 carbon atoms and whereby $B_{1,v}$ and/or $B_{2,v}$ may respectively be linked to $B_{1,v+1}$ and/or $B_{1,v-1}$ and to $B_{2,v+1}$ and/or $B_{2,v-1}$ ;

- $B_{3,v}$, for v comprised between 1 and p, refers to a hydrocarbon group possibly substituted, unsaturated and/or ramified which may contain up to 20 carbon atoms or a biguanide group or a group of formula

  (1) o-, m- or p-xylydene of formula :

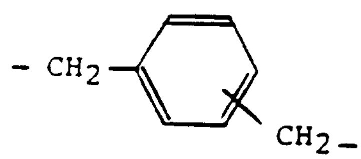

  (2)

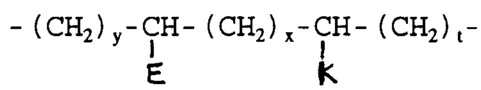

  wherein x, y and t are integers from 0 to 11, such that the sum x + y + t is equal or greater than 0 and lower to 18, while E and K refer to a hydrogen atom or to alkyl radicals containing less than 18 carbon atoms ;

  (3) $-(CH_2)_n-S-(CH_2)_n-$ ,
  (4) $-(CH_2)_n-O-(CH_2)_n-$ ,
  (5) $-(CH_2)_n-S-S-(CH_2)_n-$ ,
  (6) $-(CH_2)_n-SO-(CH_2)_n-$ ,
  (7) $-(CH_2)_n-SO_2-(CH_2)_n-$ ,
  (8)

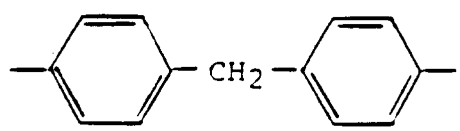

  wherein n is equal to 1,2 or 3 ;

  (9)

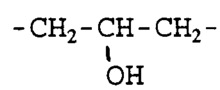

  and

$X^-_0$ and $X^-_v$ refer to an anion, preferably to a halogen atom, and at least one compound of formula

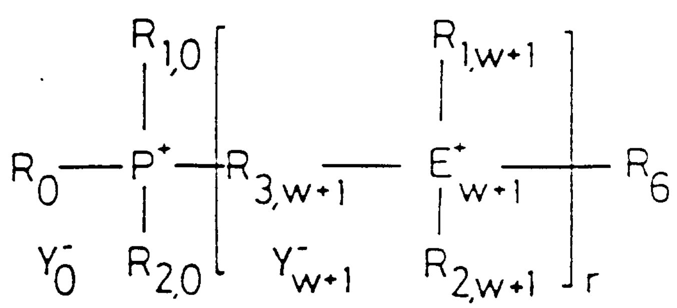

(II)

in which

- r is an integer at least equal to 0 ;
- w is an integer comprised between 0 and r ;
- $R_0$ and $R_6$ which may be identical or different refer to a hydrocarbon radical possibly substituted and possibly unsaturated, this radical containing from 1 to 22 carbon atoms ;
- $R_{1,0}$, $R_{2,0}$, $R_{1,w+1}$ and $R_{2,w+1}$, for w comprised between 0 and r, refer to a hydrocarbon group possibly ramified, unsaturated and/or substituted by one or more halogen atoms or by a hydrocarbon group or by a hydroxyl group, whereby $R_{1,w+1}$, $R_{2,w+1}$ may contain up to 22 carbon atoms and whereby $R_{1,w+1}$ and/or $R_{2,w+1}$ may respectively be linked to $R_{1,w+2}$ and/or $R_{1,w}$ and to $R_{2,w+2}$ and/or $R_{2,w}$ ;
- $E^{w+1}$ being selected for w comprised between 0 and r between N and P ;
- $R_{3,w+1}$ refers to a hydrocarbon group possibly substituted, unsaturated and/or ramified which may contain up to 20 carbon atoms or a group of formula

  (1) o-, m- or p-xylydene of formula :

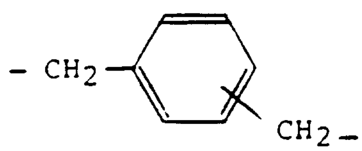

  (2)

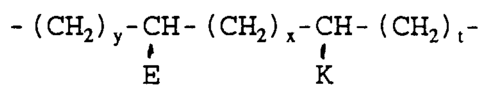

  wherein x, y and t are integers from 0 to 11, such that the sum x+y+t is equal or greater than 0 and lower to 18, while E and K refer to a hydrogen atom or to alkyl radicals containing less than 18 carbon atoms ;

  (3) $-(CH_2)_n-S-(CH_2)_n-$ ,
  (4) $-(CH_2)_n-O-(CH_2)_n-$ ,
  (5) $-(CH_2)_n-S-S-(CH_2)_n-$ ,
  (6) $-(CH_2)_n-SO-(CH_2)_n-$ ,
  (7) $-(CH_2)_n-SO_2-(CH_2)_n-$ ,
  (8)

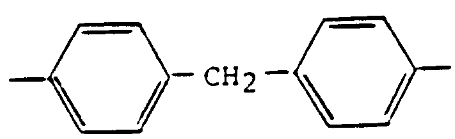

  wherein n is equal to 1,2 or 3 ;

  (9)

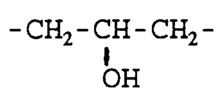

  and

$Y^-_O$ and $Y^-_{w+1}$ refer to an anion, preferably to a halogen atom.

**2.** Composition according to claim 1, characterized in that the compound of quaternary ammonium has a molecular weight comprised between 1,000 and 5,000.

**3.** Composition according to anyone of the preceding claims, characterized in that it contains also at least one ion of a metal selected among iron, copper, silver, gold, manganese, zinc or a mixture of such ions.

**4.** Composition according to anyone of the preceding claims, characterized in that it contains from 5 to 95 % by weight of compound of formula II with respect to the total weight of compound of formula I and of compound of formula II present in the composition.

**5.** Composition according to anyone of the preceding claims, characterized in that the anion is a chlorine atom or a bromine atom.

**6.** Composition according to anyone of the preceding claims, characterized in that $R_0$ and /or $R_6$ and/or $B_0$ and/or $B_6$ contain from 12 to 22 carbon atoms, preferably 16 carbon atoms.

**7.** Composition according to anyone of the preceding claims, characterized in that for at least one k comprised between 0 and r, $R_{1,k}$ and $R_{1,k+1}$ of the compound of formula II are linked together and/or $R_{2,k}$ and $R_{2,k+1}$ of the compound of formula II are linked together, forming preferably a group identical to $R_{3,k+1}$.

**8.** Composition according to anyone of the preceding claims, characterized in that for at least one l comprised between 0 and p, $B_{1,l}$ and $B_{1,l+1}$ of the compound of formula I are linked together and/or $B_{2,l}$ and $B_{2,l+1}$ of the compound of formula I are linked together, forming preferably a group identical to $B_{3,l+1}$.

**9.** Composition according to claim 7 or 8, characterized in that $R_{1,k}$ and $R_{1,k+1}$ and/or $R_{2,k}$ and $R_{2,k+1}$ and $B_{1,l}$ and $B_{1,l+1}$ and/or $B_{2,l}$ and $B_{2,l+1}$ form a group $-(CH_2)_2-$.

**10.** Composition according to claim 1, characterized in that $R_{3,k+1}$ is a group of formula $-CH_2-CH_2-CH_2-$ when k is an even number, while $R_{3,k+1}$ is a group of formula $-CH_2-CH_2-CH_2-CH_2-CH_2-CH_2-$ when k is an odd number, or inversely.

**11.** Composition according to claim 1, characterized in that $B_{3,l}$ is a group of formula $-CH_2-CH_2-CH_2-$ when l is an even number, while $B_{3,l}$ is a group of formula $-CH_2-CH_2-CH_2-CH_2-CH_2-CH_2-$ when l is an odd number, or inversely.

**12.** Composition according to claim 1, characterized in that $R_{3,k+1}$ and $B_{3,l}$ for at least one k or l comprised between 0 and r or 1 and p is a group of formula :

$$-(CH_2)_c-\underset{\displaystyle D}{\underset{|}{CH}}-(CH_2)_d-$$

in which D refers to a hydrogen atom or a $C_{1-4}$ alkyl radical, c and d are integers lower to 5, one of which may be equal to 0 while the sum c+d is at least equal to 1 and atmost equal to 8.

**13.** Process for disinfecting liquids, characterized in that at least one composition according to anyone of the preceding claims is added to said liquids.

**14.** Process for disinfecting contaminated surfaces, characterized in that said surfaces are contacted with at least one disinfecting composition according to anyone of the claims 1 to 12.

**15.** Process for preserving drinks, in which a composition according to anyone of the claims 1 to 12 is added to said drinks.

**16.** Process for preserving food, in which a composition according to anyone of the claims 1 to 12 is injected into said food.

**17.** Process for preserving food, in which said food is soaked into a composition according to anyone of the claims 1 to 12.

**Patentansprüche**

**1.** Mischung für die Desinfektion von Flüssigkeiten und Oberflächen oder die Konservierung von Lebensmitteln und Getränken, die Mischung enthält mindestens eine Quartärammonium-Verbindung mit einem Molekulargewicht zwischen 1.000 und 50.000 mit der Formel

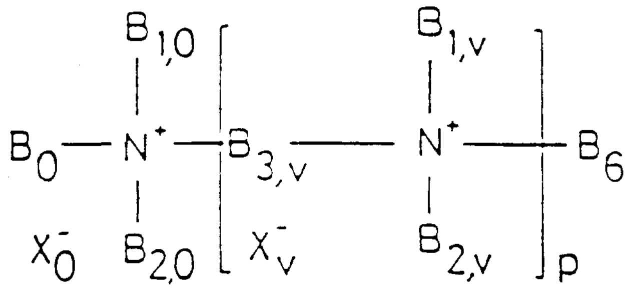

(I)

worin :

- p eine ganze Zahl größer oder gleich 1 ist ;
- v eine ganze Zahl zwischen 1 und p ist ;
- $B_0$ und $B_6$, die identisch oder verschieden sein können, ein gegebenenfalls substituiertes oder ungesättigtes Kohlenwasserstoffgruppe mit 1 bis 22 Kohlenstoffatomen bezeichnen ;
- $B_{1,0}$, $B_{2,0}$, $B_{1,v}$ und $B_{2,v}$ für v zwischen 1 und p eine gegebenenfalls verzweigte, ungesättigte und/oder mit einem oder mehrere Halogenatom(en), eine Kohlenwasserstoff-Gruppierung, eine Carboxyl-Gruppierung oder eine Hydroxyl-Gruppierung substituierte Kohlenwasserstoff-Gruppe bezeichnet, wobei $B_{1,0}$, $B_{2,0}$, $B_{1,v}$ und $B_{2,v}$ bis zu 22 Kohlenstoffatome enthalten können, und wobei $B_{1,v}$ und/oder $B_{2,v}$ jeweils an $B_{1,v+1}$ bzw. $B_{1,v-1}$ sowie an $B_{2,v+1}$ bzw. $B_{2,v-1}$ gebunden sein können ;
- $B_{3,v}$ für v zwischen 1 und p eine gegebenenfalls substituierte und/oder verzweigte Kohlenwasserstoff-Gruppe bezeichnet, die bis zu 20 Kohlenstoffatome, eine Biguanid-Gruppe oder eine Gruppe mit folgender Formel enthalten kann :

  (1) o-, m- oder p-Xylyliden mit der Formel :

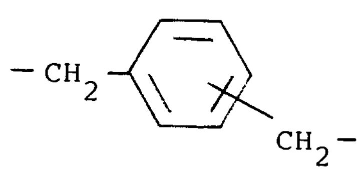

  (2)

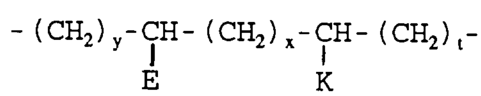

  worin x, y und t ganze Zahlen zwischen 0 und 11 sind, während die Summe aus x + y + t größer oder gleich 0 und kleiner als 18 ist, und worin E und K Wasserstoff oder Alkyl-Radikale, die unter 18 Kohlenstoffatome enthalten bezeichnen ;

  (3) $-(CH_2)_n-S-(CH_2)_n-$ ,

  (4) $-(CH_2)_n-O-(CH_2)_n-$ ,

  (5) $-(CH_2)_n-S-S-(CH_2)_n-$ ,

  (6) $-(CH_2)_n-SO-(CH_2)_n-$ ,

(7) $-(CH_2)_n-SO_2-(CH_2)_n-$ ,
(8)

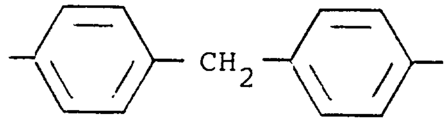

wobei n gleich 1, 2 oder 3 ist ;
(9)

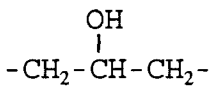

und
$X_0^-$ und $X_v^-$ ein Anion, vorzugsweise ein Halogenatom bezeichnen, und mindestens eine Verbindung mit folgender Formel

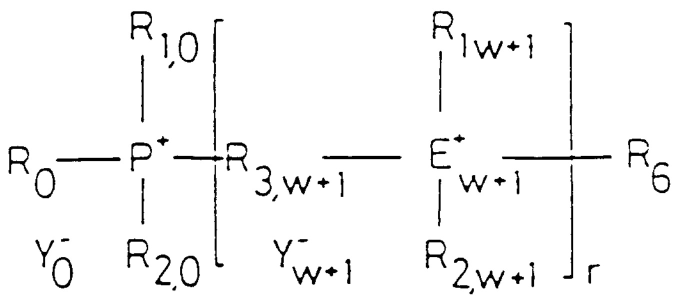 (II)

worin :
- r eine ganze Zahl mindestens gleich 0 ist ;
- w eine ganze Zahl zwischen 0 und r ist ;
- $R_0$ und $R_6$, die identisch oder verschieden sein können, ein gegebenenfalls substituiertes oder ungesättigtes Kohlenwasserstoffgruppe mit 1 bis 22 Kohlenstoffatomen bezeichnen ;
- $R_{1,0}$, $R_{2,0}$, $R_{1,w+1}$ und $R_{2,w+1}$ für w zwischen 0 und r eine gegebenenfalls verzweigte, ungesättigte und/oder durch ein oder mehrere Halogene, eine Kohlenwasserstoffgruppe oder eine Hydroxyl-Gruppierung substituierte Kohlenwasserstoff-Gruppe bezeichnen, wobei $R_{1,w+1}$ und $R_{2,w+1}$ bis zu 22 Kohlenstoffatome enthalten können, und $R_{1,w+1}$ und/oder $R_{2,w+1}$ jeweils an $R_{1,w+2}$ bzw. $R_{1,w}$ sowie an $R_{2,w+2}$ bzw. $R_{2,w}$ gebunden sein können ;
- $E^+_{w+1}$ zwischen N und P für w zwischen 0 und r ausgewählt sind ;
- $R_{3,w+1}$ eine gegebenfalls ungesättigte und/oder verzweigte Kohlenwasserstoffgruppe mit bis zu 20 Kohlenstoffatomen oder einer Gruppe mit folgender Formel enthalten kann :
(1) o-, m- oder p-Xylyliden mit der Formel :

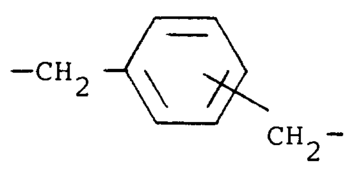

(2)

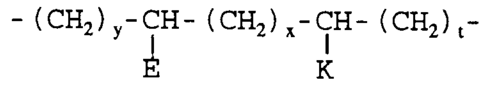

wobei x, y und t ganze Zahlen zwischen 0 und 11 sind, während die Summe aus x + y + t größer oder gleich 0 und kleiner als 18 ist, und wobei E und K Wasserstoff oder Alkyl-Radikale mit unter 18 Kohlenstoffatomen bezeichnen ;

(3) $-(CH_2)_n-S-(CH_2)_n-$ ,
(4) $-(CH_2)_n-O-(CH_2)_n-$ ,
(5) $-(CH_2)_n-S-S-(CH_2)_n-$ ,
(6) $-(CH_2)_n-SO-(CH_2)_n-$ ,
(7) $-(CH_2)_n-SO_2-(CH_2)_n-$ ,
(8)

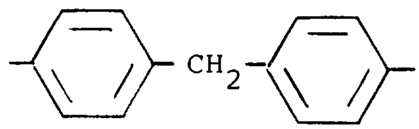

wobei n gleich 1, 2 oder 3 ist ;
(9)

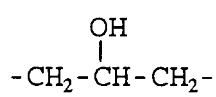

und
$Y_0^-$ und $Y_{w+1}^-$ ein Anion, vorzugsweise ein Halogenatom bezeichnen.

2. Mischung gemäß Patentanspruch 1, dadurch gekennzeichnet, daß die Quartärammonium-Verbindung ein Molekulargewicht zwischen 1000 und 5000 hat.

3. Mischung gemäß einem der vorangegangenen Patentansprüche, dadurch gekennzeichnet, daß sie ebenfalls mindestens ein ion eines Metalls, ausgewählt aus Eisen, Kupfer, Silber, Gold, Mangan, Zink oder ein Gemisch dieser Ionen enthält.

4. Mischung gemäß einem der vorangegangenen Patentansprüche, dadurch gekennzeichnet, daß sie 5 bis 95 % des Gewichts der in der Mischung enthaltenen Verbindung mit Formel II in bezug auf die Gesamtmenge der Verbindung mit Formel I und der Verbindung mit Formel II enthält.

5. Mischung gemäß einem der vorangegangenen Patentansprüche, dadurch gekennzeichnet, daß das Anion ein Chlor- oder Bromatom ist.

6. Mischung gemäß einem der vorangegangenen Patentansprüche, dadurch gekennzeichnet, daß $R_0$ und/oder $R_6$ und/oder $B_0$ und/oder $B_6$ 12 bis 22, vorzugsweise 16, Kohlenstoffatome enthalten.

7. Mischung gemäß einem der vorangegangenen Patentansprüche, dadurch gekennzeichnet, daß $R_{1,k}$ und $R_{1,k+1}$ der Verbindung mit Formel II für mindestens ein k zwischen 0 und r miteinander verbunden sind und/oder $R_{2,k}$ und $R_{2,k+1}$ der Verbindung mit Formel II miteinander verbunden sind und vorzugsweise eine mit $R_{3,k+1}$ identische Gruppe bilden.

8. Mischung gemäß einem der vorangegangenen Patentansprüche, dadurch gekennzeichnet, daß $B_{1,l}$ und $B_{1,l+1}$ der Verbindung mit Formel I für mindestens ein k zwischen 0 und p miteinander verbunden sind

und/oder $B_{2,l}$ und $B_{2,l+1}$ der Verbindung mit Formel I miteinander verbunden sind und vorzugsweise eine mit $B_{3,l+1}$ identische Gruppe bilden.

**9.** Mischung gemäß einer der Patentansprüche 7 oder 8, dadurch gekennzeichnet, daß $R_{1,k}$ und $R_{1,k+1}$ und/oder $R_{2,k}$ und $R_{2,k+1}$ und/oder $B_{1,l}$ und $B_{1,l+1}$ und/oder $B_{2,l}$ und $B_{2,l+1}$ eine $-(CH_2)_2$-Gruppe bilden.

**10.** Mischung gemäß Patentanspruch 1, dadurch gekennzeichnet, daß $R_{3,k+1}$ eine Gruppe mit der Formel $-CH_2-CH_2-CH_2-$ ist, wenn k eine gerade Zahl ist, während $R_{3,k+1}$ eine Gruppe mit Formel $-CH_2-CH_2-CH_2-CH_2-CH_2-CH_2-$ ist, wenn k eine ungerade Zahl ist, oder umgekehrt.

**11.** Mischung gemäß Patentanspruch 1, dadurch gekennzeichnet, daß $B_{3,l}$ eine Gruppe mit der Formel $-CH_2-CH_2-CH_2-$ ist, wenn l eine gerade Zahl ist, während $B_{3,l}$ eine Gruppe mit Formel $-CH_2-CH_2-CH_2-CH_2-CH_2-CH_2-$ ist, wenn l eine ungerade Zahl ist, oder umgekehrt.

**12.** Mischung gemäß Patentanspruch 1, dadurch gekennzeichnet, daß $R_{3,k+1}$ und $B_{3,l}$ für mindestens ein k oder l zwischen 0 und r oder l und p eine Gruppe mit folgender Formel ist :

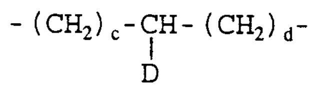

wobei D ein Wasserstoffatom oder ein Alkyl-Radikal $C_{1-4}$ bezeichnet sowie c und d ganze Zahlen unter 5 sind, von denen man einen Wert gleich 0 erhalten kann, während die Summe c + d größer oder gleich 1 und kleiner oder gleich 8 ist.

**13.** Verfahren zur Desinfektion von Flüssigkeiten, dadurch gekennzeichnet, daß man diesen Flüssigkeiten mindestens eine Mischung gemäß einem der vorangegangenen Patentansprüche beifügt.

**14.** Verfahren zur Desinfektion von infizierten Oberflächen, dadurch gekennzeichnet, daß man diese Oberflächen in Kontakt mit mindestens einer Desinfektionsmischung gemäß einem der Patentansprüche 1 bis 12 bringt.

**15.** Verfahren zur Konservierung von Getränken, bei dem man diesen Getränken eine Mischung gemäß einer der Patentansprüche 1 bis 12 hinzufügt.

**16.** Verfahren zur Konservierung von Lebensmitteln, bei dem man diesen Lebensmitteln eine Mischung gemäß einem der Patentansprüche 1 bis 12 injiziert.

**17.** Verfahren zur Konservierung von Lebensmitteln, bei dem man diese Lebensmitteln mit einer Mischung gemäß einem der Patentansprüche 1 bis 12 tränkt.